# FASCICULE DE BREVET EUROPEEN

(11) **EP 2 630 494 B1**
(45) Date de publication et mention de la délivrance du brevet: **18.03.2020**
(21) Numéro de dépôt: 11776563.6
(22) Date de dépôt: 11.10.2011
(51) Int. Cl.: G01N 33/543, G01N 33/548, C08B 37/00

(54) **PROCEDE DE FONCTIONNALISATION DE SURFACES POUR LA DETECTION D'ANALYTES**
VERFAHREN ZUR FUNKTIONALISIERUNG VON OBERFLÄCHEN FÜR DEN ANALYTNACHWEIS
METHOD FOR FUNCTIONALISING SURFACES FOR ANALYTE DETECTION

(30) Priorité: 28.03.2011 FR 1152558; 18.10.2010 FR 1058469
(43) Date de publication de la demande: 28.08.2013
(73) Titulaire: Centre National de la Recherche Scientifique, 75794 Paris Cedex 16 (FR); Innobiochips, 59000 Lille (FR); Université de Lille 2 Droit et Santé, 59000 Lille (FR); Université de Lille 1 Sciences et Technologies, 59655 Villeneuve d'Ascq Cedex (FR)
(72) Inventeur: MELNYK, Oleg, 59112 Annoeullin (FR); EBRAN, Jean-Philippe, Georges, Bernard, 59000 Lille (FR); DHEUR, Julien, Philippe, 59480 La Bassee (FR); DENDANE, Nabil, 75116 Paris (FR); SOUPLET, Vianney, 59233 Maing (FR); OLIVIER, Christophe, 59113 Seclin (FR)
(74) Mandataire: Hirsch & Associés
(86) Numéro de dépôt international: PCT/IB2011/054472
(87) Numéro de publication internationale: WO 2012/052874

(56) Documents cités:
- EP-A2- 0 146 455
- WO-A1-2005/095507
- FR-A1- 2 794 649

### SEQUENCE LISTING

<110> Centre national de la recherche scientifique Innobiochips université Lille 1 sciences et technologies Université Lille 2
<120> Procédé de fonctionnalisation de surfaces pour la détection d'analytes
<110> 31389
<160> 9
<170> RatentIn version 3.5
<210> 1
   <211> 2
   <212> PRT
   <213> Artificial sequence
<220>
   <223> obtained by synthesis
<220>
   <221> MISC_FEATURE
   <222> (1)..(1)
   <223> Rhodamine group at the N-terminal end
<220>
   <221> MISC_FEATURE
   <222> (2) .. (2)
   <223> NH(CH2)3-NH-CHOCO
<400> 1
   Lys Arg
   1
<210> 2
   <211> 2
   <212> PRT
   <213> Artificial Sequence
<220>
   <223> Obtained by synthesis
<220>
   <221> MISC_FEATURE
   <222> (1)..(1)
   <223> Rhodamine group at the N-terminal end
<220>
   <221> MISC_FEATURE
   <222> (2)..(2)
   <223> NH2 group at the C-terminal end
<400> 2
   Lys Arg
   1
<210> 3
   <211> 32
   <212> PRT
   <213> Artificial Sequence
<220>
   <223> Obtained by synthesis
<220>
   <221> MISC_FEATURE
   <222> (32)..(32)
   <223> NH2 group at the C-terminal end
<400> 3
   Ser Gly Tyr Pro Tyr Asp Val Pro Asp Tyr Ala Gly Tyr Pro Tyr Asp
   1 5 10 15
   Val Pro Asp Tyr Ala Gly Tyr Pro Tyr Asp Val Pro Asp Tyr Ala Ser
   20 25 30
<210> 4
   <211> 26
   <212> PRT
   <213> Artificial Sequence
<220>
   <223> obtained by synthesis
<220>
   <221> MISC_FEATURE
   <222> (26)..(26)
   <223> NH2 group at the c-terminal end
<400> 4
   Ser Asp Tyr Lys Asp His Asp Gly Asp Tyr Lys Asp His Asp Ile Asp
   1 5 10 15
   Tyr Lys Asp Asp Asp Asp Lys Gly Gly Ser
   20 25
<210> 5
   <211> 31
   <212> PRT
   <213> Artificial sequence
<220>
   <223> Obtained by synthesis
<220>
   <221> MISC_FEATURE
   <222> (1)..(1)
   <223> CHOCO group at the N terminal end
<220>
   <221> MISC_FEATURE
   <222> (31)..(31)
   <223> NH2 group at the C-termmal end
<400> 5
   Gly Tyr Pro Tyr Asp Val Pro Asp Tyr Ala Gly Tyr Pro Tyr Asp Val
   1 5 10 15
   Pro Asp Tyr Ala Gly Tyr Pro Tyr Asp Val Pro Asp Tyr Ala Ser
   20 25 30
<210> 25
   <212> PRT
   <213> Artificial Sequence
<220>
   <223> Obtained by synthesis
<220>
   <221> MISC_FEATURE
   <222> (1)..(1)
   <223> CHOCO group at the N terminal end
<220>
   <221> MISC_FEATURE
   <222> (25)..(25)
   <223> NH2 group at the C-terminal end
<400> 6
<210> 7
   <211> 10
   <212> PRT
   <213> Artificial Sequence
<220>
   <223> obtained by synthesis
<220>
   <221> MISC_FEATURE
   <222> (4)..(4)
   <223> Rhodamine
<220>
   <221> MISC_FEATURE
   <222> (10)..(10)
   <223> NH2 group at the C-terminal end
<400> 7
   Cys Ile Leu Lys Glu Pro Val His Gly Val
   1               5                   10
<210> 8
   <211> 10
   <212> PRT
   <213> Artificial Sequence
<220>
   <223> Obtained by synthesis
<220>
   <221> MISC_FEATURE
   <222> (4)..(4)
   <223> Rhodamine
<220>
   <221> MISC_FEATURE
   <222> (10)..(10)
   <223> NH2 group at the C-terminal end
<400> 8
   Ser Ile Leu Lys Glu Pro Val His Gly Val
   1               5                   10
<210> 9
   <211> 9
   <212> PRT
   <213> Artificial Sequence
<220>
   <223> Obtained by synthesis
<220>
   <221> MISC_FEATURE
   <222> (3)..(3)
   <223> Mtt
<220>
   <221> MISC_FEATURE
   <222> (4)..(4)
   <223> TBu
<220>
   <221> MISC_FEATURE
   <222> (7)..(7)
   <223> Trt
<220>
   <221> MISC_FEATURE
   <222> (9)..(9)
   <223> NH2 group at the C terminal end
<400> 9
   Ile Leu Lys Glu Pro Val His Gly Val
   1               5

## Description

### DOMAINE DE L'INVENTION

La présente invention concerne un procédé de fonctionnalisation de surfaces pour la détection d'analytes. Ce procédé de fonctionnalisation de surfaces permet notamment d'obtenir des dispositifs de détection d'analytes qui peuvent être utilisés pour mettre en œuvre divers tests chimiques, biologiques et diagnostiques. L'invention fournit également des polymères particuliers qui sont adaptés à la mise en œuvre dudit procédé de fonctionnalisation de surfaces.

### ARRIERE-PLAN TECHNIQUE

Le test de référence en diagnostic est le test dit « ELISA ». Ce test permet de détecter ou de doser un analyte dans un liquide biologique. En général, les puits d'une plaque de microtitration sont tapissés (par interaction essentiellement hydrophobe) avec un élément de capture (par exemple un anticorps) capable de lier spécifiquement un analyte (notamment un antigène) recherché. La solution à tester est ensuite déposée dans les puits de la microplaque, et si la molécule recherchée est présente, elle se lie spécifiquement à l'élément de capture.

Un élément de traçage, également capable de se lier à la molécule recherchée, est ajouté dans les puits. Cet élément de traçage peut être par exemple une enzyme catalysant la formation d'un produit coloré, de sorte que cet élément de traçage puisse être quantifié par colorimétrie.

Le test « ELISA » est bien adapté à l'automatisation. Toutefois, les surfaces classiquement utilisées pour ce test (en polystyrène) ne permettent pas d'immobiliser facilement des molécules hydrophiles, comme les polysaccharides chargés négativement, ou de faible poids moléculaire. Aussi, il a par exemple été proposé de conjuguer un polysaccharide à une polylysine afin de pouvoir le fixer à une surface de polystyrène, en utilisant les capacités d'adsorption de la polylysine (Leinonen & Frasch, Infect Immun., 38(3):1203-7, 1982).

Le test « ELISA » présente également des limitations liées à la charge de surface, du fait que les interactions sont généralement plus spécifiques avec des surfaces chargées négativement (Graves, J. Immunol. Methods, 111(2):157-66, 1988), alors que les surfaces de plastique sont hydrophobes.

En outre le test « ELISA » standard est limité aux analyses mono-paramétriques, c'est-à-dire qu'une seule information est disponible par test et par échantillon. Lorsque plusieurs analyses sont nécessaires sur le même échantillon, il est nécessaire d'effectuer plusieurs tests de type « ELISA » de préférence en parallèle, soit de manière classique, soit de façon miniaturisée, dans un dispositif connu sous le nom de « biopuce ». Très peu de biopuces sont aujourd'hui disponibles sur le marché, et les dispositifs proposés à l'heure actuelle utilisent principalement des supports de lame de microscope en verre. Un tel support convient difficilement à une utilisation de masse.

D'un autre côté, les supports plastiques se prêtent mal à la fabrication de biopuces. Des rapports signal / bruit insatisfaisants sont généralement obtenus en adsorption directe sur le support, du fait de l'importante adsorption non-spécifique des analytes. Les surfaces plastiques hydrophobes ont aussi l'inconvénient de dénaturer les protéines, et ne peuvent donc pas être utilisées sans modification.

Toutefois les possibilités de fonctionnalisation des matières plastiques par modification chimique des polymères concernés sont très limitées, peu de groupements chimiques pouvant être introduits. En outre, un changement mineur de la composition des polymères, voire des variations de composition de lot à lot, conduiraient à des différences de propriétés des substrats fonctionnalisés.

Par ailleurs, des contraintes importantes sont rencontrées lors de l'impression de biopuces, car il est difficile de déposer des nanogouttes de façon reproductible (de sorte à obtenir des dépôts bien définis et réguliers) sur de tels supports. La méthode la plus pratique pour déposer des gouttes dans ce contexte, l'impression contact à aiguilles, est un défi technique, si bien que la technique de dépôt non contact de type piézoélectrique est généralement utilisée. Cependant, étant donné sa lenteur, cette dernière permet difficilement une production industrielle.

Quelques exemples de fonctionnalisation de plaques à 96 puits en plastique ont été proposés. Ainsi, la technique plasma a été suggérée dans Marson, Robinson et al., Glycobiology 19(2):1537-46 (2009), pour la préparation de puces à sucre. Cette technique pose la difficulté de conserver la fonctionnalité du sucre après immobilisation. Un autre exemple repose sur l'utilisation de la streptavidine pour revêtir les surfaces plastiques (Gehring, Albin et al., Anal. Bioanal. Chem., 5 avril 2008).

Le document EP 1 953 555 décrit un dispositif comprenant un substrat en matière plastique recouvert par un film métallique sur lequel sont immobilisés une substance physiologiquement active et un composé permettant de créer des liaisons hydrogène.

Le document EP 2 030 677 décrit un biocapteur comprenant un substrat recouvert par un film métallique à travers duquel est fixé un polymère anionique.

Ces documents ne divulguent pas une interaction directe entre le polymère et un substrat en matière plastique.

Il existe donc encore un besoin de fournir des dispositifs de détection d'analytes faciles à fabriquer, robustes, faciles à utiliser, et / ou permettant une détection de nombreux analytes en parallèle.

### RESUME DE L'INVENTION

L'invention concerne en premier lieu un dispositif de détection d'analytes, comprenant un substrat en matière plastique recouvert au moins en partie par des polymères de liaison fixés au substrat de manière non-covalente, lesdits polymères de liaison comportant un squelette polysaccharidique pourvu :
- de groupements aromatiques de la forme -X-CONH-Z, où X représente une chaîne alkyle linéaire ou ramifiée, substituée ou non, comprenant de 1 à 6 atomes de carbone, et Z représente une fonction aryle ; et
- de groupements acides carboxyliques de la forme -X-COOH, où X représente une chaîne alkyle linéaire ou ramifiée, substituée ou non, comprenant de 1 à 6 atomes de carbone ;
ledit dispositif étant caractérisé en ce que le squelette polysaccharidique est en outre pourvu de groupements réactifs F, lesdits groupements réactifs étant de la forme -X-CONH-Z', où X représente une chaîne alkyle linéaire ou ramifiée, substituée ou non, comprenant de 1 à 6 atomes de carbone, et Z' représente un groupement apte à se lier à une autre molécule, Z' étant choisi parmi :
-X'-N₃ ;
-NH₂ ; 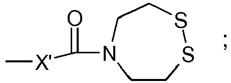 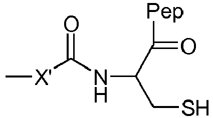 où X' représente une chaîne alkyle, substituée ou non, comprenant de 1 à 6 atomes de carbone et Pep représente un fragment peptidique.

Selon un mode de réalisation, le squelette polysaccharidique est un squelette de dextran, dont le poids moléculaire est de préférence compris entre 15 000 et 100 000, et de manière plus particulièrement préférée entre 30 000 et 60 000.

Selon un mode de réalisation, les groupements acides carboxyliques sont de la forme -X-COOH, où X est CH₂.

Selon un mode de réalisation, les groupements aromatiques sont de la forme -X-CONH-Z, où X est CH₂, et Z représente une fonction aryle comprenant de 6 à 30 atomes de carbone, Z étant de préférence une fonction benzyle éventuellement substituée, telle que -CH₂-Ph ou -CH₂-Ph-paraOH.

Selon un mode de réalisation, les polymères de liaison comportent :
- de 0,4 à 0,8 groupements aromatiques, de préférence de 0,4 à 0,6 groupements aromatiques, de préférence de 0,45 à 0,6 groupements aromatiques par unité saccharidique du squelette polysaccharidique; et / ou

- de 0 à 0,8 groupements réactifs, de préférence de 0 à 0,6 groupements réactifs, par unité saccharidique du squelette polysaccharidique ; et / ou
- de 0,5 à 1,5 groupements aromatiques, acides carboxyliques et réactifs au total, de préférence de 0,9 à 1,3 groupements aromatiques, acides carboxyliques et réactifs au total, par unité saccharidique du squelette polysaccharidique.

Selon un mode de réalisation, le substrat est un substrat de polystyrène, polycarbonate, polyméthacrylate de méthyle ou polypropylène, et de préférence de polystyrène.

Selon un mode de réalisation, le dispositif comporte des éléments de capture immobilisés sur les polymères de liaison, lesdits éléments de capture étant de préférence choisis parmi les polypeptides éventuellement modifiés et/ou conjugués, les saccharides, oligosaccharides ou lipopolysaccharides, les virus ou fragments de virus et les cellules, l'immobilisation des éléments de capture sur les polymères de liaison étant de préférence réalisée par adsorption ou par liaison covalente aux groupements réactifs F.

Selon un mode de réalisation, le dispositif comporte une pluralité de zones de détection, les zones de détection comportant de préférence des éléments de capture différents.

Selon un mode de réalisation, le substrat est une lame opaque ou transparente, une plaque de microtitration, un ensemble de billes, une plaque de culture, une bandelette ou un bâtonnet.

Il est également décrit un polymère comportant un squelette polysaccharidique pourvu de groupements aromatiques, de groupements acides carboxyliques ainsi que de groupements réactifs de formule (V) :

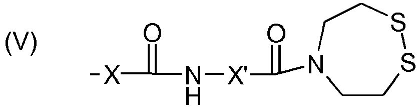

X et X' représentant chacun une chaîne alkyle, substituée ou non, comportant de 1 à 6 atomes de carbone.

Il est également décrit un polymère comportant un squelette polysaccharidique pourvu de groupements aromatiques, de groupements acides carboxyliques ainsi que de groupements réactifs de formule (V') :

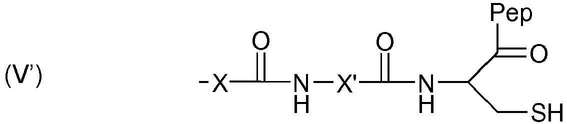

X et X' représentant chacun une chaîne alkyle, substituée ou non, comportant de 1 à 6 atomes de carbone et Pep représentant un fragment peptidique.

L'invention a également pour objet un polymère de liaison susceptible d'être utilisé dans le dispositif décrit ci-avant, comportant un squelette polysaccharidique pourvu :
- de 0,4 à 0,8 groupements aromatique par unité saccharidique de la forme -X-CONH-Z, où X représente une chaîne alkyle linéaire ou ramifiée, substituée ou non, comprenant de 1 à 6 atomes de carbone, et Z représente une fonction aryle ;
- des groupements acides carboxyliques de la forme -X-COOH, où X représente une chaîne alkyle linéaire ou ramifiée, comprenant de 1 à 6 atomes de carbone,
- de groupements réactifs F de la forme -X-CONH-Z', où X représente une chaîne alkyle linéaire ou ramifiée, substituée ou non, comprenant de 1 à 6 atomes de carbone, et Z' est choisi parmi :
   -X'-N₃ ;
   -NH₂ ; 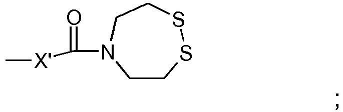 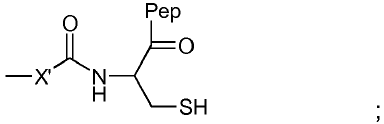 où X' représente une chaîne alkyle, substituée ou non, comprenant de 1 à 6 atomes de carbone et Pep représente un fragment peptidique.

Selon un mode de réalisation des deux polymères ci-dessus, X représente CH₂ et / ou X' représente (CH₂)₂.

Il est également décrit un procédé de fabrication du polymère ci-dessus doté de groupements réactifs de formule (V) : 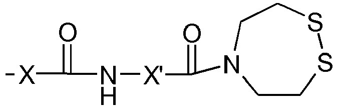 ledit procédé comprenant :
- la fourniture d'un polysaccharide ;
- le greffage de groupements acides carboxyliques sur le polysaccharide ; puis
- la modification d'une partie des groupements acides carboxyliques greffés pour fournir des groupements aromatiques ; suivie ou précédée de
- la modification d'une autre partie des groupements acides carboxyliques greffés pour fournir des groupements réactifs de formule (V),
ladite modification comprenant la réaction du polysaccharide avec le composé de formule (VII) : 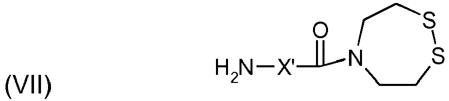 dans laquelle X' représente une chaîne alkyle, substituée ou non, comportant de 1 à 6 atomes de carbone.

Il est également décrit un procédé de fabrication du polymère ci-dessus doté de groupements réactifs de formule (V'),
ledit procédé comprenant :
- la fourniture d'un polymère de formule (V) ci-dessus ; et
- la réaction de ce polymère avec le polypeptide H-Cys-Pep, où Pep représente un fragment peptidique et Cys représente le résidu cystéine.

Selon un mode de réalisation du dispositif décrit ci-dessus, les polymères de liaison sont des polymères susmentionnés (dotés de groupements réactifs de formule (V) ou (V')).

L'invention a également pour objet l'utilisation d'un dispositif de détection d'analytes tel que décrit ci-dessus, pour la détection et éventuellement la quantification de molécules chimiques, de molécules biologiques, de cellules ou d'organismes vivants.

Il est également décrit un procédé de fabrication d'un dispositif de détection d'analytes tel que décrit ci-dessus, comprenant :
- la fourniture d'un substrat en matière plastique ;
- la mise en contact du substrat avec au moins une solution comprenant des polymères de liaison.

Selon un mode de réalisation, ce procédé comprend en outre la mise en contact du substrat avec une ou plusieurs solutions comprenant des éléments de capture.

Il est également décrit un procédé de fabrication d'un dispositif de détection d'analytes tel que décrit ci-dessus, comprenant :
- la fourniture d'un substrat en matière plastique ;
- la mise en contact du substrat avec une ou plusieurs solutions comprenant des polymères de liaison liés à des éléments de capture.

La présente invention permet de surmonter les inconvénients de l'état de la technique. Elle fournit plus particulièrement des dispositifs de détection d'analytes faciles à fabriquer, robustes et faciles à utiliser. Selon un mode de réalisation, ces dispositifs permettent une détection de nombreux analytes en parallèle. En particulier, l'invention permet d'immobiliser des molécules hydrophiles et / ou de petite taille (mais également des anticorps ou protéines).

Ceci est accompli grâce à une fonctionnalisation de surface au moyen de polymères de liaison présentant un squelette polysaccharidique. Les polymères de liaison comportent des groupements aromatiques, qui favorisent la fixation non-covalente des polymères de liaison à des surfaces relativement hydrophobes, ainsi que des groupements acides carboxyliques, permettant de charger négativement les polymères de liaison et ainsi de limiter les phénomènes d'adsorption non-spécifique entre les polymères de liaison et les analytes à détecter (qui sont eux-mêmes souvent chargés négativement).

L'utilisation de surfaces en plastique et notamment en polystyrène dans les dispositifs de l'invention permet d'éviter les problèmes liés au verre : risque de bris de verre et danger associé pour le personnel, coût lié à l'élimination des déchets, hétérogénéité de fonctionnalisation liée à la nature chimique du verre, coût élevé du substrat et de la caractérisation, difficulté d'effectuer une compartimentation sur le substrat.

Selon certains modes de réalisation particuliers, l'invention présente également une ou de préférence plusieurs des caractéristiques avantageuses énumérées ci-dessous.
- Les polymères de liaison sont facilement synthétisés en grandes quantités et peuvent être caractérisés par des techniques analytiques classiques (RMN, pH-métrie, microanalyse), ce qui rend possible un contrôle rigoureux de la fabrication.
- Les polymères de liaison sont fixés sur les surfaces par simple adsorption (liaison non-covalente), au moyen notamment des groupements aromatiques des polymères de liaison. Il s'agit d'un mode de fixation qui est particulièrement simple à mettre en œuvre, robuste, et peu sensible à des variations modérées des propriétés physicochimiques des surfaces. Il est de toute façon également possible d'ajuster la densité des groupements aromatiques dans les polymères de liaison, en fonction de la nature des surfaces.
- L'invention peut être mise en œuvre à partir de substrats largement disponibles actuellement et peu onéreux : plaques de microtitration en polystyrène, boîtes de Pétri etc.
- L'invention permet également de réaliser des biopuces, c'est-à-dire des dispositifs de détection d'analytes miniaturisés permettant une détection en parallèle d'un grand nombre d'analytes, à partir de supports en plastique et notamment en polystyrène.
- La fonctionnalisation des surfaces selon l'invention est effectuée en fixant des éléments de capture (ou sondes) sur les polymères de liaison, soit de façon covalente (par l'intermédiaire de groupements réactifs présents sur les polymères de liaison), notamment lorsque les éléments de capture sont des molécules de petite taille, soit encore de façon non-covalente (par adsorption). Ainsi, l'invention présente une grande flexibilité de mise en œuvre.
- Les groupements fonctionnels sont avantageusement stables (conservent leurs propriétés) en présence d'humidité ou d'air.

### BREVE DESCRIPTION DES FIGURES

La **figure** 1 représente la relation entre le degré de substitution en groupements aromatiques (en ordonnée) dans le polymère **PsAcAr1** de l'exemple 3, par rapport au nombre d'équivalents de benzylamine (en abscisse). On se rapportera à l'exemple 4 à ce sujet.
La **figure 2** représente la relation entre le degré de substitution en groupements azides (en ordonnée) dans le polymère **PsAcF1** de l'exemple 7, par rapport au nombre d'équivalents d'amine azide (en abscisse). On se rapportera à l'exemple 8 à ce sujet.
La **figure 3** est un histogramme représentant des mesures d'angles de goutte (en degrés) sur des plaques de polystyrène fonctionnalisées ou non. On se reportera à l'exemple 22 à ce sujet.
Les **figures 4, 5** et **6** illustrent la spécificité de fixation de la concanavaline A (par rapport à d'autres lectines) sur un polymère selon l'invention. On se reportera aux exemples 23 et 24 à ce sujet.
La **figure 7** est un histogramme illustrant l'influence du taux de substitution aromatique sur l'angle de goutte (en degrés) sur des plaques de polystyrène fonctionnalisées. On se reportera à l'exemple 25 à ce sujet.
La **figure 8** est un histogramme illustrant la liaison chimiosélective d'un peptide aldéhyde à une surface fonctionnalisée avec un polymère selon l'invention. On se reportera à l'exemple 26 à ce sujet.
Les **figures 9** et **10** sont des histogrammes représentant la fonctionnalisation de surfaces avec des peptides et la détection par des anticorps appropriés (incubation avec un anticorps anti-HA ou anti-FLAG). On se reportera à l'exemple 27 à ce sujet.
La **figure 11** est un histogramme illustrant la liaison chimiosélective d'un peptide comportant une cystéine N-terminale à une surface fonctionnalisée avec un polymère selon l'invention. On se reportera à l'exemple 33 à ce sujet.
Les **figures 12** et **13** sont des graphiques illustrant une détection colorimétrique sur une biopuce à anticorps au fond de plaques à 96 puits en polystyrène. On se reportera à l'exemple 34 à ce sujet.

### DESCRIPTION DE MODES DE REALISATION DE L'INVENTION

L'invention est maintenant décrite plus en détail et de façon non limitative dans la description qui suit.

### Polymères de liaison et fabrication de ceux-ci

Les polymères de liaison utilisés dans le cadre de l'invention comportent un squelette polysaccharidique (abréviation « Ps » dans la suite) pourvu de groupements acides carboxyliques (abréviation « Ac » dans la suite) de groupements aromatiques (abréviation « Ar » dans la suite) et de groupements réactifs distincts des groupements acides carboxyliques susmentionnés (abréviation « F » dans la suite).

Par « squelette polysaccharidique » on entend une structure formée par un ensemble de sucres (ou unités saccharidiques), liés entre eux par des liaisons O-osidiques.
Cette structure peut être linéaire ou ramifiée. Elle comprend de préférence de 80 à 600 unités saccharidiques, et de manière plus particulièrement préférée de 150 à 350 unités saccharidiques.

Les unités saccharidiques sont de préférence cycliques. Il peut s'agir de trioses, tétraoses, pentoses, hexoses ou heptoses, et de préférence ce sont des hexoses.

On appelle « taux de substitution » ou « degré de substitution » le nombre moyen de substituant (Ac, Ar ou F) par unité saccharidique. Des méthodes de détermination des taux de substitution sont fournies dans la section exemples.

L'ensemble des groupements Ac, Ar et F sont de préférence greffés sur tout ou partie des fonctions hydroxyles des unités saccharidiques.

Le poids moléculaire du squelette polysaccharidique est de préférence compris entre 15 000 et 100 000, et de manière plus particulièrement préférée entre 30 000 et 60 000.

Le squelette polysaccharidique peut être de type homopolyosidique (unités saccharidiques identiques) ou hétéropolyosidique (unités saccharidiques différentes).

A titre d'exemples d'homopolyosides, on peut citer : les glucosanes ou glucanes (alpha-glucanes ou beta-glucanes), tels que l'amidon, le glycogène, la cellulose, le dextran, le pullulane, l'acide hyaluronique, la chitine ou le chitosan (forme déacétylée de la chitine) ; les arabanes, les xylanes et les pectines. A titre d'exemples d'hétéropolyosides, on peut citer les gommes qui sont des structures ramifiées comportant du D-galactose, du L-arabinose, du L-rhamnose, et de l'acide D-glucuronique, ainsi que les hémicelluloses.

Selon un mode de réalisation préféré, les unités saccharidiques sont des unités glucoses (ou dextroses). Et de préférence, le squelette polysaccharidique est une molécule de dextran, c'est-à-dire qu'il présente une chaîne principale de glucoses reliés par des liaisons glycosidiques α-1,6 avec éventuellement des ramifications rattachées à la chaîne principale par des liaisons α-1,2 et / ou α-1,3 et/ou α-1,4. Le dextran est disponible en grande quantité, peu onéreux et bien soluble dans l'eau.

Dans la suite de la description, on se référera à un squelette polysaccharidique de type dextran, étant entendu que cette description s'applique de manière analogue aux autres types de squelettes polysaccharidiques.

Ne font pas partie de l'invention les polymères de liaison qui ne sont pas pourvus des groupements réactifs F (polymères de type « PsAcAr »). Ces polymères de liaison peuvent être obtenus en :
- fournissant un polysaccharide Ps (squelette polysaccharidique non substitué) ;
- greffant des groupements Ac sur ce polysaccharide de sorte à obtenir un polymère de type « PsAc » ;
- modifiant une partie des groupements Ac du polymère PsAc pour obtenir le polymère PsAcAr.

Ainsi, en faisant référence à la formule générale (I) ci-dessous : 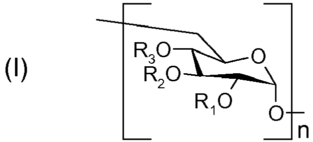 le polymère Ps de départ est un polymère de formule (I) dans laquelle l'ensemble des groupements R₁, R₂ et R₃ représentent un atome d'hydrogène ; le polymère intermédiaire PsAc est un polymère de formule (I) dans laquelle une partie des groupements R₁, R₂ et R₃ représentent un atome d'hydrogène et l'autre partie des groupements R₁, R₂ et R₃ représentent un groupement portant une fonction acide carboxylique ; et le polymère de liaison PsAcAr est un polymère de formule (I) dans laquelle une partie des groupements R₁, R₂ et R₃ représentent un atome d'hydrogène, une autre partie des groupements R₁, R₂ et R₃ représentent un groupement portant une fonction acide carboxylique et la troisième et dernière partie des groupements R₁, R₂ et R₃ représentent un groupement portant un noyau aromatique.

Les groupements acides carboxyliques Ac sont de la forme -X-COOH, où X représente une chaîne alkyle linéaire ou ramifiée comprenant de 1 à 6 atomes de carbone, éventuellement pourvue d'un ou plusieurs substituants choisis parmi le chlore, le brome, un groupement cétone, le fluor, un groupement alcool, un groupement acide carboxylique et un groupement aromatique (notamment phényle). De préférence, cette chaîne est linéaire et non substituée et / ou comprend au plus 5 atomes de carbone, au plus 4 atomes de carbone, au plus 3 atomes de carbone ou au plus 2 atomes de carbone. Selon un mode de réalisation préférée, dans lequel d'éventuels problèmes d'excès de réactivité sont évités, les groupements Ac sont de la forme -CH₂-COOH (groupements acides méthylcarboxyliques).

Il est entendu que les groupements Ac peuvent être sous forme ionisée (COOH remplacé par COO⁻ associé à un contre-ion) ou sous forme salifiée (COOH remplacé par exemple par COONa) selon le contexte.

De préférence les groupements Ac sont tous identiques, mais il est également possible de prévoir un greffage de différents groupements Ac.

Les groupements aromatiques Ar sont de la forme -Y-Z où Y représente un groupement de liaison et Z une fonction aryle, de préférence comprenant de 6 à 30 atomes de carbone, de préférence de 6 à 24 atomes de carbone, plus particulièrement de 6 à 12 atomes de carbone, et un ou plusieurs substituants choisis parmi les halogènes, -OH, -NH₂, -NO₂, -OR', -COOR', -CONHR' où R' est un groupement alkyle comportant de 1 à 6 atomes de carbone. Z peut être monocyclique ou polycyclique, et éventuellement hétérocyclique. De préférence Z est un dérivé benzénique, et notamment un groupement phényle, benzyle ou phénol.

Y est un groupement amide de la forme -X-CONH-, où X a la même signification qu'exposé ci-dessus en relation avec les groupements Ac.

Selon un mode de réalisation préféré, les groupements Ar sont des groupements -CH₂-CONH-CH₂-Ph (où Ph est un groupement phényle) ou des groupements -CH₂-CONH-CH₂-Ph-*para*OH.

De préférence les groupements Ar sont tous identiques, mais il est également possible de prévoir un greffage de différents groupements Ar.

L'étape de greffage des groupements Ac sur le polysaccharide Ps pour obtenir le polymère PsAc peut être effectuée en faisant réagir le polysaccharide Ps avec un composé acide carboxylique comportant une fonction halogène, et de préférence une fonction chlore.

Par exemple, le greffage des groupements Ac préférés de forme -CH₂-COOH peut être obtenu en faisant réagir le polysaccharide Ps avec l'acide monochloroacétique. De préférence, la réaction est effectuée en présence d'isopropanol.

L'étape de modification d'une partie des groupements Ac du polymère PsAc pour obtenir le polymère PsAcAr peut être effectuée en faisant réagir le polymère PsAc avec un composé de type R-Z où Z a la même signification que ci-dessus, et R est une fonction réactive avec la fonction carboxylique. De préférence, R est une fonction amine, apte à établir une liaison amide avec les groupements Ac.

Par exemple, les groupements Ar préférés du type -CH₂-CONH-CH₂-Ph peuvent être obtenus en faisant réagir le polysaccharide PsAc avec de la benzylamine. Par exemple encore, les groupements Ar préférés du type -CH₂-CONH-CH₂-Ph-*para*OH peuvent être obtenus en faisant réagir le polysaccharide PsAc avec de la *para*-hydroxybenzylamine. De préférence, on effectue la réaction en présence de 1-cyclohexyl-3-(2-morpholinoéthyl)carbodiimide.

Les polymères de liaison selon l'invention qui sont pourvus de groupements réactifs F (polymères de type « PsAcArF ») peuvent être obtenus à partir des polymères de type PsAc (tels qu'obtenus ci-dessus), en modifiant une partie des groupements Ac d'une part en groupements F et d'autre part en groupements Ar. Ces deux étapes de modification peuvent être effectuées dans n'importe quel ordre.

Autrement dit, on peut d'abord produire un polymère de type PsAcAr à partir d'un polymère PsAc (ainsi que cela a été décrit ci-dessus), puis modifier ce polymère de type PsAcAr pour obtenir le polymère de type PsAcArF. Et on peut également produire d'abord un polymère de type « PsAcF » à partir d'un polymère PsAc, puis modifier ce polymère de type PsAcF pour obtenir le polymère de type PsAcArF (la méthode utilisée pour cette deuxième modification étant la même que celle utilisée pour modifier le polymère de type PsAc en polymère de type PsAcAr).

L'avantage de ces différentes stratégies est de permettre de moduler les taux de substitution selon que l'on souhaite un taux plus élevé en groupements Ar, ou un taux plus élevé en groupement F.

En faisant référence à la formule générale (I) ci-dessous : 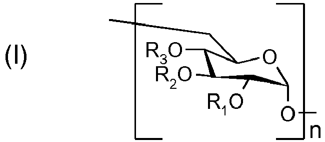 le polymère PsAcF est un polymère de formule (I) dans laquelle une partie des groupements R₁, R₂ et R₃ représentent un atome d'hydrogène, une autre partie des groupements R₁, R₂ et R₃ représentent un groupement acide carboxylique et la troisième et dernière partie des groupements R₁, R₂ et R₃ représentent un groupement réactif (différent du groupement acide carboxylique) ; et le polymère PsAcArF est un polymère de formule (I) dans laquelle une partie des groupements R₁, R₂ et R₃ représentent un atome d'hydrogène, une autre partie des groupements R₁, R₂ et R₃ représentent un groupement acide carboxylique, une autre partie des groupements R₁, R₂ et R₃ représentent un groupement aromatique et la quatrième et dernière partie des groupements R₁, R₂ et R₃ représentent un groupement réactif (différent du groupement acide carboxylique).

Les groupements réactifs F sont de la forme -Y-Z' où Y représente un groupement de liaison (même signification que ci-dessus) et Z' représente un groupement apte à se lier, notamment de manière covalente, avec un élément de capture, tel que décrit plus en détail ci-dessous. Selon un mode de réalisation préféré, le groupement Z' est apte à établir une liaison covalente avec un polypeptide.

Y est un groupement amide de la forme -X-CONH-, où X a la même signification qu'exposé ci-dessus en relation avec les groupements Ac (et X est de préférence CH₂).

Selon un premier mode de réalisation préféré, les groupements Z' sont des groupements azides (et notamment alkylazides), et les groupements F sont de préférence des groupements de la forme -X-CONH-X'-N₃ où X et X' représentent chacun une chaîne alkyle, substituée ou non, comportant de 1 à 6 atomes de carbone, les groupements F peuvent donc par exemple être des groupements - CH₂-CONH-(CH₂)₃-N₃. De tels groupements sont particulièrement utiles en tant que groupements intermédiaires, permettant d'obtenir par exemple des groupements de type triazole (voir le troisième mode de réalisation ci-dessous). Ils peuvent également lier certains éléments de capture.

Selon un deuxième mode de réalisation préféré, les groupements Z' sont des groupements hydrazides, et les groupements F sont de préférence des groupements de la forme -X-CONH-NH₂ où X représente une chaîne alkyle, substituée ou non, comportant de 1 à 6 atomes de carbone, les groupements F peuvent donc par exemple être des groupements -CH₂-CONH-NH₂. Ces groupements F sont susceptibles de se lier avec une fonction aldéhyde de peptides par exemple. Ils peuvent également favoriser l'adsorption de polypeptides (immobilisation non covalente).

Il est également décrit que les groupements Z' sont des groupements triazoles, de préférence substitués par une ou plusieurs chaînes alkyles linéaires ou ramifiées, substituées ou non, comprenant de 1 à 6 atomes de carbone, et les groupements F peuvent donc par exemple être des groupements de formule (II) : 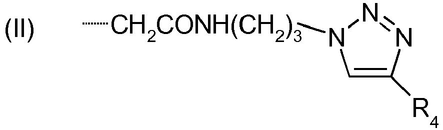 dans laquelle R₄ représente le groupement de formule (III) :

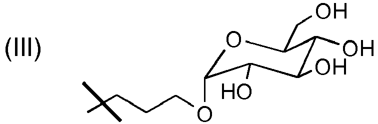

Ce type de groupements F permet de lier certains éléments de capture particuliers. Les groupements F de formule (II) ci-dessus où R₄ représente-(CH₂)₃OH sont utilisés comme témoins négatifs dans les exemples.

Selon un troisième mode de réalisation préféré, les groupements Z' sont de formule (IV) suivante : 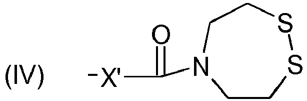 dans laquelle X' représente une chaîne alkyle linéaire ou ramifiée comprenant de 1 à 6 atomes de carbone, éventuellement pourvue de substituants halogènes (notamment Cl ou Br). De préférence X' est (CH₂)₂. Selon ce troisième mode de réalisation, les groupements F sont donc de préférence de formule (V) suivante : 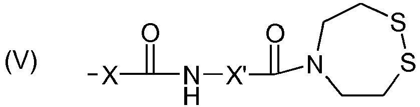 dans laquelle X et X' ont la signification mentionnée ci-dessus. Selon un mode particulièrement préféré, X représente CH₂ et X' représente (CH₂)₂.

Les groupements F correspondant à ce quatrième mode de réalisation peuvent réagir avec les molécules portant une fonction beta-amino thiol ou gamma-amino thiol, comme par exemple les polypeptides présentant une cystéine ou une homocystéine en position N-terminale, pour former une liaison amide. Ainsi les polymères de liaison correspondants à ce quatrième mode de réalisation peuvent se lier à des éléments de capture formés par des polypeptides présentant une cystéine ou une homocystéine en position N-terminale.

Ainsi, dans le cas d'une cystéine, on obtient un polymère de liaison, dans lequel les groupements F liés aux éléments de capture sont de formule (V') : 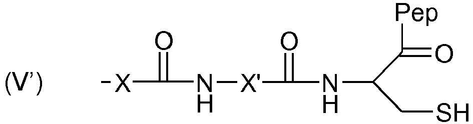 X et X' ayant la signification ci-dessus et Pep représentant un fragment peptidique, c'est-à-dire une portion de polypeptide comprenant au moins un résidu d'acide aminé (de préférence une succession de plusieurs résidus d'acides aminés), pourvue d'une fonction C-terminale (par exemple COOH ou CONH₂).

Les groupements F peuvent être tous identiques, ou bien on peut prévoir un greffage de différents groupements F.

L'étape de modification d'une partie des groupements Ac du polymère PsAc (respectivement du polymère PsAcAr) pour obtenir le polymère PsAcF (respectivement le polymère PsAcArF) peut être effectuée en faisant réagir le polymère PsAc (respectivement le polymère PsAcAr) avec un composé de type R-Z' où Z' a la même signification que ci-dessus, et R est une fonction réactive avec la fonction carboxylique. De préférence, R est une fonction amine, apte à établir une liaison amide avec les groupements Ac.

Certains groupements F peuvent être greffés en greffant d'abord des groupements réactifs F' intermédiaires, puis en modifiant chimiquement les groupements F' pour donner les groupements F à l'issue d'une étape de réaction distincte.

Par exemple, les groupements F du type -CH₂-CONH-NH₂ peuvent être obtenus en faisant réagir le polysaccharide PsAc (ou le polymère PsAcAr) avec de l'hydrazine (de préférence en présence de 1-cyclohexyl-3-(2-morpholinoéthyl)carbodiimide.

Les groupements F du type -CH₂-CONH-(CH₂)₃-N₃ peuvent être obtenus en faisant réagir le polysaccharide PsAc (ou le polymère PsAcAr) avec du chlorhydrate de 3-azidopropylammonium (de préférence en présence de 1-cyclohexyl-3-(2-morpholinoéthyl)carbodiimide.

Les groupements F de formule (II) ci-dessus peuvent être obtenus en faisant réagir un polymère PsAcF (ou un polymère PsAcArF) pourvu de groupements réactifs du type -CH₂-CONH-(CH₂)₃-N₃ avec le composé de formule (VI) : 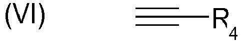 (de préférence en présence d'ions cuivre (II) et d'ascorbate de sodium).

Dans ce cas de figure, on obtient un polymère PsAcF (ou un polymère PsAcArF) dans lequel soit la totalité des groupements F sont de formule (II) ci-dessus, soit une partie des groupements F sont de formule (II) ci-dessus et une partie des groupements F sont du type -CH₂-CONH-(CH₂)₃-N₃ (en cas de substitution incomplète par les composés de formule (VI)).

Les groupements F de formule (V) ci-dessus peuvent être obtenus en faisant réagir le polysaccharide PsAc (ou le polymère PsAcAr) avec le composé de formule (VII) : 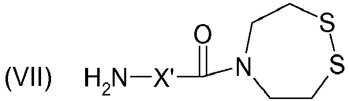 (de préférence en présence de 1-cyclohexyl-3-(2-morpholinoéthyl)carbodiimide) dans laquelle X' a la signification mentionnée ci-dessus. Le composé de formule (VII) peut être fabriqué comme indiqué dans les exemples ci-dessous.

De manière générale, le taux de substitution en Ac, Ar et F peut être ajusté en fonction d'une part de l'ordre dans lequel on effectue les greffages de groupements (Ac puis Ar puis F ou bien Ac puis F puis Ar), et d'autre part en adaptant les conditions de greffage et notamment le rapport molaire entre le polysaccharide et le réactif responsable du greffage du groupement considéré.

Des polymères dérivés de dextrans et répondant à la formule générale des polymères de liaison ci-dessus, avec pour groupements réactifs F des groupements sulfates ou sulfonates, ainsi que l'utilisation à des fins thérapeutiques de ces polymères, ont été décrits dans les documents WO 99/29734, WO 00/76452, WO 00/76562, WO 01/91742 et FR 2891149.

De préférence, les polymères de liaison de l'invention sont dépourvus de fonctions sulfates ou sulfonates. En effet de telles fonctions sont susceptibles d'interagir avec de nombreuses protéines, ce qui est néfaste pour une détection optimale. En outre, l'absence de fonctions sulfates ou sulfonates est particulièrement utile lorsque les éléments de capture sont eux-mêmes sulfatés (oligosaccharides en particulier).

### Fonctionnalisation de surfaces

Les polymères de liaison selon l'invention (de type PsAcArF) peuvent être déposés sur un substrat afin de fonctionnaliser la surface de ce substrat. Le substrat est en matière plastique (matière solide de nature polymérique, en général façonnée à chaud et sous pression) qui de préférence est choisie parmi le polystyrène, le polycarbonate, le polyméthacrylate de méthyle et le polypropylène.

Ainsi, l'invention permet par exemple de fonctionnaliser la surface de lames en plastique transparent ou opaque, ou bien de plaques de microtitration en polystyrène (par exemple à 96 puits), de billes (magnétiques ou non), de plaques de culture en polystyrène (par exemple à 96 puits), de bandelettes ou de bâtonnets en polystyrène.

La fonctionnalisation comprend en général deux étapes : une première étape de fixation des polymères de liaison à la surface du substrat et une deuxième étape de fixation d'éléments de capture sur les polymères de liaison. Alternativement, on peut lier les polymères de liaison aux éléments de capture avant de fixer les polymères de liaison à la surface du substrat (particulièrement lorsque la liaison entre les polymères de liaison et les éléments de capture est de type covalent).

La fixation des polymères de liaison à la surface du substrat s'effectue, notamment pour des surfaces planes, en trempant la surface à traiter dans une solution comprenant le polymère PsAcAr ou PsAcArF. Pour les surfaces moulées pouvant contenir un liquide (par exemple les plaques de titration à 96 puits), la fonctionnalisation peut être effectuée par simple remplissage. On prévoit généralement ultérieurement un lavage et séchage. En général, la solution de polymère est à une concentration de 0,1 à 50 µg/mL, et de préférence de 1 à 10 µg/mL.

Sans vouloir être liés par une quelconque théorie, les inventeurs estiment que les polymères de liaison se fixent de manière non-covalente à la surface du substrat (adsorption) notamment par l'intermédiaire des groupements Ar.

Les éléments de capture fixés sur les polymères de liaison peuvent être des polypeptides éventuellement modifiés et / ou conjugués, des saccharides, oligosaccharides ou lipopolysaccharides, des virus ou fragments de virus ou même des cellules. Par exemple les éléments de capture peuvent être des anticorps.

Le terme « polypeptide », dans le cadre de la présente demande, recouvre toute chaîne de résidus d'acides aminés (en nombre supérieur ou égal à deux) reliés par des liaisons peptidiques. Les « polypeptides » au sens de la présente demande peuvent donc par exemple être des oligopeptides, peptides ou protéines selon l'acception conventionnelle de ces termes. Les résidus d'acides aminés présents dans les polypeptides selon l'invention peuvent être choisis parmi les résidus d'acides aminés protéinogéniques ou non. De préférence, ils sont choisis parmi les vingt résidus d'acides aminés protéinogéniques.

La fixation des éléments de capture aux polymères de liaison peut intervenir par adsorption si l'on utilise des polymères de liaison sans groupements réactifs F. Elle peut également intervenir par établissement de liens covalents avec les groupements réactifs F. Elle peut encore intervenir par adsorption favorisée par des groupements réactifs F. C'est par exemple le cas pour l'adsorption d'anticorps favorisée par des groupements de type hydrazide.

Il est également possible de prévoir des éléments de capture comprenant plusieurs parties. Par exemple, on peut utiliser un élément de capture intermédiaire (par exemple un polypeptide ou un composé saccharidique ou un glycopeptide) susceptible de se fixer aux polymères de liaison, et un élément de capture final (par exemple un virus ou un fragment de virus ou une cellule) susceptible de se lier à l'élément de capture intermédiaire.

Lorsque le dispositif de détection d'analytes est destiné à la détection unique d'un analyte (ou d'un type d'analytes), il suffit de déposer les polymères de liaison (et le ou les éléments de capture) sur l'ensemble de la surface utile du substrat.

Lorsque le dispositif de détection d'analytes est destiné à détecter plusieurs analytes avec des éléments de capture distincts, plusieurs zones distinctes de détection doivent être ménagées. Les plaques de microtitration à 96 puits sont un exemple de substrat particulièrement utile à cet effet.

Dans ce cas, on peut soit déposer les polymères de liaison sur l'ensemble de la surface utile du substrat, puis déposer les divers éléments de capture sur des zones délimitées du substrat (par exemple par dépôt de petites gouttes de diverses solutions contenant les éléments de capture sur des emplacements déterminés du substrat) ; soit déposer différentes solutions de polymères de liaison liés aux éléments de capture directement sur des zones délimitées du substrat (par exemple dans les puits d'une plaque de microtitration) ; soit encore déposer une ou plusieurs solutions de polymères de liaison directement sur des zones délimitées du substrat (par exemple dans les puits d'une plaque de microtitration) puis déposer différentes solutions contenant les éléments de capture sur les mêmes zones délimitées du substrat.

Les zones de la surface du substrat recouvertes de polymères de liaison sont de préférence hydrophiles, caractérisées par un angle de goutte de l'eau inférieur ou égal à 70°, de préférence inférieur ou égal à 65° et idéalement inférieur ou égal à 60°. L'hydrophilie de la surface du substrat recouverte de polymères de liaison permet de minimiser les interactions non-spécifiques de nombreux analytes (notamment protéines) avec le substrat. Elle permet également le dépôt de nanogouttes pour la préparation de biopuces, alors que ce type de dépôt est complexe et peu reproductible quand il est réalisé directement sur des substrats en plastique non traités.

Les analytes susceptibles d'être détectés grâce à l'invention peuvent être toutes sortes de matériaux chimiques ou biologiques : particules minérales, molécules organiques (notamment pesticides ou autres polluants), biomolécules (notamment composés saccharidiques, polypeptides, modifiés ou non, conjugués ou non), virus ou fragments de virus, cellules ou organismes (unicellulaires ou multicellulaires).

En particulier, l'invention permet de fabriquer des puces à polypeptides, utiles pour le sérotypage, le criblage d'épitopes, la quantification de protéines dans les milieux biologiques, ou encore l'analyse de relations entre molécules peptidiques du type ligand-récepteur.

De manière générale, l'utilisation des dispositifs de détection selon l'invention implique la mise en contact de la surface du substrat revêtue des polymères de liaison et des éléments de capture avec une ou plusieurs solutions ou suspensions susceptibles de comprendre les analytes d'intérêt (par exemple échantillon d'eau issue de l'environnement, échantillon de produit alimentaire, échantillon biologique tel que l'urine, le sang ou un produit dérivé du sang, etc.).

La détection des analytes éventuellement fixés de manière spécifique aux éléments de capture peut être effectuée au moyen d'éléments de traçage, qui peuvent être par exemple fluorescents, radioactifs ou marqués chimiquement, et sont susceptibles de se lier aux analytes retenus sur le dispositif selon l'invention ou de réagir avec eux d'une autre manière. Ensuite, les éléments de traçage retenus sur le dispositif selon l'invention peuvent être identifiés et éventuellement quantifiés au moyen d'un appareil de détection de fluorescence, colorimétrique ou de détection de radioactivité.

Il est également possible de se fonder sur une modification du milieu (par exemple une coloration visible à l'œil nu) due à une réaction chimique entre l'élément de traçage et l'analyte, ou bien entre l'élément de traçage (fixé à l'analyte) et un réactif supplémentaire.

A titre d'illustration, l'élément de traçage peut comprendre une enzyme catalysant la formation d'un produit coloré. Il peut également s'agir de billes revêtues de molécules susceptibles de se lier aux analytes retenus. On peut également utiliser des anticorps marqués (fluorescents ou radioactifs) susceptibles de se fixer sur des antigènes présents sur les analytes.

La détection et éventuellement la quantification des analytes rendues possibles par l'invention trouvent notamment à s'appliquer dans le diagnostic médical (pour la médecine humaine ou vétérinaire).

### EXEMPLES

Différents polymères avec un squelette polysaccharidique de dextran sont préparés dans les exemples qui suivent :
- le polymère **PsAc1** qui ne fait pas partie de l'invention, comprend des groupements Ac de type -CH₂COONa ;
- le polymère **PsAcAr1** qui ne fait pas partie de l'invention, comprend des groupements Ac de type -CH₂COONa et des groupements Ar de type - CH₂CONHCH₂Ph ;
- le polymère **PsAcF1** qui ne fait pas partie de l'invention, comprend des groupements Ac de type -CH₂COONa et des groupements F de type - CH₂CONH(CH₂)₃N₃ ;
- le polymère **PsAcArF1** qui comprend des groupements Ac de type -CH₂COONa, des groupements Ar de type -CH₂CONHCH₂Ph et des groupements F de type -CH₂CONHNH₂ ;
- le polymère **PsAcArF2** qui comprend des groupements Ac de type -CH₂COONa, des groupements Ar de type -CH₂CONHCH₂Ph et des groupements F de type -CH₂CONH(CH₂)₃N₃ ;
- le polymère **PsAcF2** qui ne fait pas partie de l'invention, comprend des groupements Ac de type -CH₂COONa et des groupements F de formule (II) ci-dessus, avec R₄ représentant -(CH₂)₃OH ;
- le polymère **PsAcF3** qui ne fait pas partie de l'invention, comprend des groupements Ac de type -CH₂COONa et des groupements F de formule (II) ci-dessus, avec R₄ ayant la formule (III) ci-dessus ;
- le polymère **PsAcArF3** qui ne fait pas partie de l'invention, comprend des groupements Ac de type -CH₂COONa, des groupements Ar de type -CH₂CONHCH₂Ph et des groupements F de formule (II) ci-dessus, avec R4 représentant -(CH₂)₃OH ;
- le polymère **PsAcArF4** qui ne fait pas partie de l'invention, comprend des groupements Ac de type -CH₂COONa, des groupements Ar de type -CH₂CONHCH₂Ph et des groupements F de formule (II) ci-dessus, avec R₄ ayant la formule (III) ci-dessus ;
- le polymère **PsAcArF5** qui comprend des groupements Ac de type -CH₂COONa, des groupements Ar de type -CH₂CONHCH₂Ph et des groupements F de formule (V) ci-dessus, avec X représentant CH₂ et X' représentant (CH₂)₂.

On note sous la forme PsAc (x) un polymère comprenant un taux de substitution en groupements Ac égal à x.

On note sous la forme PsAcAr (x ; y) un polymère comprenant un taux de substitution total en groupements Ac et Ar égal à x et un taux de substitution en groupements Ar égal à y.

On note sous la forme PsAcF (x ; z) un polymère comprenant un taux de substitution total en groupements Ac et F égal à x et un taux de substitution en groupements F égal à z.

On note sous la forme PsAcArF (x ; y ; z) un polymère comprenant un taux de substitution total en groupements Ac, Ar et F égal à x, un taux de substitution en groupements Ar égal à y et un taux de substitution en groupements F égal à z.

### Exemple 1 - préparation du polymère PsAc1 (protocole général)

Du dextran T40 (2 g, 11,1 mmol, 1 eq) est dissous dans 6,4 mL d'eau deionisée. On ajoute lentement à cette solution de l'isopropanol (36 mL) sous très forte agitation, puis de la soude (2,4 g, 60 mmol, 5,4 eq), et le mélange réactionnel est agité pendant 1 heure à 60°C. Enfin on ajoute de l'acide monochloroacétique (3 g, 31,7 mmol), puis on agite une nuit à 60°C. La pâte blanche obtenue est récupérée et précipitée trois fois dans du méthanol et centrifugée. Le solide obtenu est dissous dans de l'eau déionisée et lyophilisé pour fournir le polymère **PsAc1.** Typiquement, ce protocole permet d'obtenir un taux de substitution des groupements Ac de 1,2 à 1,4.

### Exemple 2 - mesure du taux de substitution en groupements acides carboxyliques

La détermination du taux de substitution en groupements methylcarboxylates est effectuée par RMN 1H après hydrolyse.

Pour cela, le produit est hydrolysé comme suit :
Le polymère **PsAc1** (200 mg) est solubilisé dans 2 mL de D₂O, puis on ajoute lentement 666 µL de D₂SO₄. Le mélange réactionnel est chauffé à 90°C pendant 4 heures. Le mélange est analysé par RMN 1H 300 MHz.

Le degré de substitution (DS) est calculé selon l'équation suivante : DS = A/B, avec A : (1/2) × intégrale du signal des protons du CH₂CO₂H entre 4 et 4,5 ppm ; et B : intégrale des protons portés les carbones C₁ des unités glucose, ou (1/6) × intégrale des protons portés par les carbones C₂₋₆ entre 3 et 4 ppm.

### Exemple 3 - préparation du polymère PsAcAr1

On dissout 100 mg de PsAc1 (1,1) dans 2 mL d'eau deionisée, puis on ajuste le pH à 4,74 avec une solution de HCl à 1N. On ajoute du 1-cyclohexyl-3-(2-morpholino-ethyl)carbodiimide (CMC) (233 mg, 0,55 mmol, 1,1 eq), puis on ajuste de nouveau le pH à 4,74 avec une solution de HCl à 1N. On ajoute de la benzylamine et le mélange réactionnel est agité pendant 1 nuit à température ambiante. Les échantillons sont ensuite dialysés pendant 70 heures dans une solution de NaCl à 2 M puis 100 heures dans de l'eau deionisée pour fournir le polymère **PsAcAr1.**

### Exemple 4 - mesure du taux de substitution en groupements aromatiques

Le degré de substitution en groupements aromatiques est calculé grâce aux spectres RMN 1H 300 MHz selon l'équation suivante: DS=(B/A)/5, avec A: l'intégrale des protons portés par C₁ ; B : l'intégrale des protons aromatiques dus au groupement phényle.

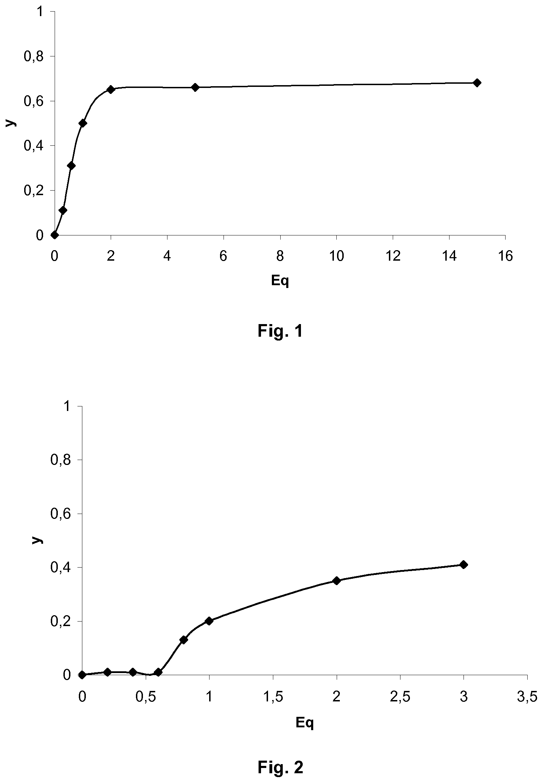

La **figure 1** illustre la relation entre le degré de substitution (y) en groupement CH₂CONHCH₂Ph et le nombre d'équivalents de benzylamine (eq) engagés dans la réaction (0,3 eq, 0,6 eq, 1 eq, 2 eq, 5 eq et 15 eq par rapport au taux de substitution en groupements carboxyliques disponibles).

### Exemple 5 - synthèse de 2-(3-azidopropyl)-isoindole-1,3-dione

Du *N*-(3-bromopropyl)phtalimide (15 g, 56,02 mmol) est dissous dans 200 mL de diméthylformamide, puis on ajoute de l'azoture de sodium (7,25 g, 112,05 mmol, 2 eq). L'agitation est maintenue pendant 2 heures à 70°C. Le milieu réactionnel est ensuite évaporé à sec puis co-évaporé avec du toluène pour éliminer les traces de diméthylformamide. Le résidu obtenu est solubilisé dans de l'éther diéthylique (150 mL), et la phase organique est lavée avec de l'eau (2 x 150 mL). La phase organique est séchée sur du sulfate de magnésium anhydre. Le solvant est évaporé pour donner de la 2-(3-azidopropyl)-isoindole-1,3-dione sous forme d'une poudre blanche (12,44 g, 54,1 mmol, 97%).

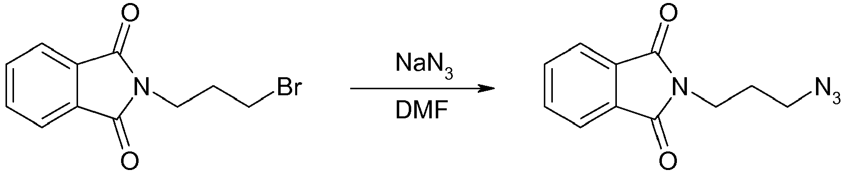

RMN-¹H (CDCl₃, 300 MHz) : *δ* ppm 7,78 (m, 2H), 7,65 (m, 2H), 3,72 (t, 2H, *J* = 6,9 Hz), 3,31 (t, 2H, *J* = 6,9 Hz), 1,90 (tt, 2H, *J* = 6,9 Hz).
RMN-¹³C (75 MHz CDCl₃) : *δ* ppm 168,28, 134,04, 131,96, 123,31, 49,0, 35,35, 28,0 ; MALDI-TOF : M_{calculé} = 230,08 (C₁₁H₁₀N₄O₂), trouvé (*m*/*z*) 231,1 [M + H]⁺, 253,1 [M + Na]⁺.

### Exemple 6 - synthèse de 3-azidopropylamine

On dissout de la 2-(3-azidopropyl)-isoindole-1,3-dione (3 g, 12 mmol) dans 10 mL de dichlorométhane, puis on ajoute de l'hydrazine (9,6 mL, 32 mmol, 2,6 eq). Le mélange réactionnel est chauffé à reflux pendant 1 nuit sous agitation. On ajoute alors une solution aqueuse d'acide chlorhydrique à 1N (100 mL). La phase organique est décantée puis la phase aqueuse est lavée avec du dichlorométhane (3 x 50 mL). Le pH de la phase aqueuse est ajusté à 14 avec une solution saturée de soude. La phase aqueuse est alors extraite avec du dichlorométhane (3 x 40 mL). Les phases dichlorométhane sont rassemblées et extraites avec une solution aqueuse d'acide chlorhydrique 1N (3 x 50 mL). La solution aqueuse acide est finalement congelée et lyophilisée. On obtient du chlorhydrate de 3-azidopropylammonium sous la forme d'une poudre blanche (1,49 g, 10,97 mmol, 84 %).

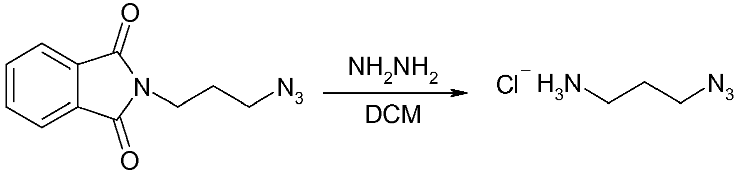

RMN-¹H (D₂O, 300 MHz) : *δ* ppm 3,52 (t, 2H, *J* = 6,6 Hz), 3,11 (t, 2H, *J =* 7,2 Hz), 1,96 (tt, 2H, *J* = 6,6 Hz).
RMN-¹³C (D₂O75 MHz) : *δ* ppm 39,02, 26,48, 15,67.
MALDI-TOF : M_{calculé} = 100,07 (C₃H₈N₄) : trouvé m/z 101,0 [M+H]⁺, 123,0 [M + Na]⁺, 139,0 [M + K]⁺, 107,1 [M+Li]⁺.

### Exemple 7 - préparation du polymère PsAcF1

On dissout 100 mg de **PsAc1 (1,4)** dans 2 mL d'eau deionisée, puis on ajuste le pH à 4,74 avec une solution de HCl à 1N. On ajoute ensuite du 1-cyclohexyl-3-(2-morpholino-ethyl)carbodiimide (CMC) (296,5 mg, 0,7 mmol, 1,1 eq) et le pH est ajusté à nouveau à 4,74 avec une solution de HCl à 1N. On ajoute la 3-azidopropylamine sous forme de chlorhydrate, puis le pH est ajusté à 8,4. Le mélange réactionnel est agité pendant 1 nuit à température ambiante. Les échantillons sont ensuite dialysés pendant 70 heures dans une solution de NaCl à 2 M puis 100 heures dans de l'eau deionisée. Pour finir, le produit **PsAcF1** ainsi obtenu est congelé et lyophilisé.

### Exemple 8 - mesure du taux de substitution en groupements réactifs

Le degré de substitution en groupements azides est calculé selon l'équation suivante : DS=(B/A)/2, avec A : l'intégrale des protons portés par les carbones C₁ ; B : l'intégrale des protons dans le champ fort (due aux signaux des protons de la 3-azidopropylamine).

La **figure 2** illustre la relation entre le degré de substitution en groupements azides (y) et le nombre d'équivalents de 3-azidopropylamine (Eq) engagés dans la réaction, par rapport au taux de substitution en groupements carboxyliques disponibles.

### Exemple 9 - préparation du polymère PsAc1 (1,15)

A une solution de 5 g (27,7 mmol, 1 eq) de dextran T40 dissous dans 16 mL d'eau est ajouté 90 mL d'isopropanol goutte à goutte sous une forte agitation. 6 g (150 mmol, 5,4 eq) de soude sont ajoutés au milieu réactionnel qui est alors laissé sous agitation à 60°C pendant 1 h.

A ce mélange est ensuite ajouté 7,5 g (79,36 mmol, 2,8 eq) d'acide monochloroacétique, Ce dernier est alors laissé sous agitation à 60°C pendant une nuit. On obtient une pate blanchâtre qui est solubilisée dans 50 mL d'eau. Le produit est alors précipité dans 500 mL de MeOH refroidi à 0°C sous forte agitation. Les culots obtenus après centrifugation (2500 tours/min, 10 min, 4°C) sont solubilisés dans le minimum d'eau et le produit est précipité une seconde fois dans 500 mL de MeOH. Les culots, obtenus après la seconde précipitation, sont lavés deux fois au MeOH puis solubilisés dans le minium d'eau et lyophilisés. On obtient 3 g (15,7 mmol) de **PsAc1** avec un degré de substitution de 1,15, c'est-à-dire **PsAc1 (1,15).**

### Exemple 10 - préparation du polymère PsAcAr1 (1,15 ; 0,64)

Le pH d'une solution contenant 468 mg (2,44 mmol, 1 eq) de **PsAc1 (1,15)** dissous dans 9,4 mL d'eau est ajusté à 4,74 par ajout de HCl à 1N et 0,1N. A cette solution est ajouté 1,187 g (2,806 mmol) de CMC, le pH est de nouveau ajusté à 4,74. 266 µL (2,44 mmol, 1 eq par rapport à x) de benzylamine sont alors ajoutés au milieu réactionnel qui est laissé sous agitation magnétique pendant une nuit à température ambiante. Le pH final de la solution est de 10,2.

Le milieu réactionnel est alors introduit dans un boudin de dialyse (3500 g/mol ; 1 mL/cm) qui est plongé dans 3,5 L d'une solution de NaCl à 2M pour y rester pendant 5 jours puis dans 3,5 L d'eau pour y rester également pendant 5 jours.

Le contenu du boudin est alors mis dans un pot afin d'être congelé puis lyophilisé. On obtient 477mg (1,67 mmol) de **PsAcAr1 (1,15 ; 0,64)** (taux de substitution en groupement benzylamino de 0,64).

### Exemple 11 - préparation du polymère PsAcArF1 (1,15 ; 0,64 ; 0,26)

Le pH d'une solution contenant 256 mg (0,895 mmol, 1 eq) de **PsAcAr1 (1,15 ; 0,64)** dissous dans 2 ml d'eau est ajusté à 4,74 par ajout de HCl à 1N et 0,1N. A cette solution est ajouté 212,5 mg (0,502 mmol) de CMC, le pH est de nouveau ajusté à 4,74. 870 µL (4,56 mmol) d'une solution d'hydrazine à 24-26% dans l'eau sont alors ajoutés au milieu réactionnel qui est laissé sous agitation magnétique pendant une nuit à température ambiante. Le pH final de la solution est de 10,28.

Le milieu réactionnel est ensuite introduit dans un boudin de dialyse (3500 g/mol ; 1 mL/cm) qui est plongé dans 3,5 L d'une solution de NaCl à 2M pour y rester pendant 5 jours puis dans 3,5 L d'eau pour y rester également pendant 5 jours.

Le contenu du boudin est alors mis dans un pot afin d'être congelé et lyophilisé. On obtient ainsi 235 mg (0.81 mmol) de **PsAcArF1 (1,15 ; 0,64 ; 0,26)** (taux de substitution en hydrazide de 0,26).

### Exemple 12 - préparation du polymère PsAcAr1 (1,2 ; 0,49)

Le pH d'une solution de 513,3 mg de **PsAc1 (1,2),** dissous dans 10 mL d'eau deionisée, est ajusté à 4,74 à l'aide d'une solution d'acide chlorhydrique à 1 N. Du N-Cyclohexyl-*N'*-(2-morpholinoethyl)-carbodiimide méthylsulfonate (931,85 mg, 2,2 mmoles, 1,1 eq.) est ajouté à température ambiante, puis le pH de la solution est ajusté à 4,74 de la même manière. De la benzylamine (428,6 mg, 4,0 mmoles, 2 eq.) est ensuite ajoutée goutte à goutte et la solution est agitée à température ambiante pendant 16 heures. Le milieu réactionnel est purifié par dialyse dans une solution de NaCl à 2 M pendant 3 jours, puis dans de l'eau deionisée pendant 3 jours. Après lyophilisation, on obtient le **PsAcAr1 (1,2 ; 0,49)** sous forme d'une poudre amorphe blanche (degré de substitution de 0,49 en groupements benzylamino).
RMN-¹H (D₂O, 300 MHz) : δ (ppm) : 7,21 (s, Is, Hₐᵣ), 5,09 (s, Is, Hₐₙₒ), 4,89 (s, Is, Hₐₙₒ), 4,53-3,39 (m, Is).

### Exemple 13 - préparation du polymère PsAcF1 (1,2 ; 0,38)

513,3 mg de **PsAc1 (1,2)** sont dissous dans 10 mL d'eau déionisée. Le pH de la solution est ajusté à 4,74 à l'aide d'une solution de HCl 1 N. Du *N*-Cyclohexyl-*N*'-(2-morpholinoethyl)-carbodiimide méthylsulfonate (931,85 mg, 2,2 mmoles, 1,1 eq.) est ajouté à la solution et le pH est de nouveau ajusté à 4,74. Du chlorhydrate de 3-azidopropylammonium (819,5 mg, 6 mmoles, 3 eq.) est introduit au milieu réactionnel, puis le pH est ajusté à 8,4 grâce à une solution de Na₂CO₃ à 1 N. La réaction est agitée à température ambiante pendant 16 heures. Le produit brut est dialysé pendant 3 jours dans une solution de NaCl à 2 M puis 3 jours dans de l'eau déionisée. La lyophilisation permet l'obtention du **PsAcF1 (1,2 ; 0,38)** sous forme d'un poudre blanche amorphe (degré de substitution en groupements azido de 0,38).
RMN-¹H (D₂O, 300 MHz) : δ (ppm) : 5,07 (s, Is, Hₐₙₒ), 4,88 (s, Is, Hₐₙₒ), 4,28-3,26 (m, Is), 1,77 (s, Is, CH₂).

### Exemple 14 - préparation du polymère PsAcArF2 (1,2 : 0,34 : 0,38)

Une solution de 272,2 mg de **PsAcF1 (1,2 ; 0,38)** et dissous dans 5 mL d'eau déionisée est ajustée à pH 4.74 avec une solution d'HCl 1 N. Le *N*-Cyclohexyl-*N*'-(2-morpholinoethyl)-carbodiimide méthylsulfonate (372,8 mg, 0,88 mmoles, 1,1 eq.) est introduit et le pH du milieu réactionnel est ajusté de nouveau à 4,74 avec une solution d'HCl à 1N. La benzylamine (171,4 mg, 4,6 mmoles, 2 eq.) est additionnée et la solution est mélangée à température ambiante pendant 18 heures. La dialyse dans du NaCl à 2 N pendant 3 jours, puis dans de l'eau déionisée pendant 3 jours, suivie de la lyophilisation permet l'obtention du composé **PsAcArF2 (1,2 ; 0,34** ; **0,38)** (degré de substitution de 0,34 en groupement benzylamino).
RMN-¹H (D₂O, 300 MHz) : δ (ppm) : 7,23 (s, Is, Hₐᵣ), 5,05 (s, Is, Hₐₙₒ), 4,84 (s, Is, Hₐₙₒ), 4,33-3,26 (m, Is), 1,68 (s, Is, CH₂).

### Exemple 15 - préparation du polymère PsAcArF2 (1,2 ; 0,49 : 0,62)

A une solution de 224,2 mg de **PsAcAr1 (1,2; 0,49)** dans 5 mL d'eau déionisée à pH=4,74, est ajouté le *N*-Cyclohexyl-*N*'-(2-morpholinoethyl)-carbodiimide méthylsulfonate (372,7 mg, 0,88 mmoles, 1,1 eq.). Le pH de la solution est ajusté à 4,74 avec une solution d'HCl à 1N puis le chlorhydrate de 3-azidopropylammonium (327,8 mg, 2,4 mmoles, 3 eq.) est ajouté. Le pH est ajusté à 8,4 puis la solution est agitée à température ambiante pendant 17 heures. La dialyse dans du NaCl à 2 N pendant 3 jours, puis dans de l'eau déionisée pendant 3 jours, et enfin la lyophilisation, donne le polymère **PsAcArF2 (1,2; 0,49 ; 0,62)** (degré de substitution de 0,62 en groupement azide).
RMN-¹H (D₂O, 300 MHz) : δ (ppm) : 7,23 (s, Is, Hₐᵣ), 5,05 (s, Is, Hₐₙₒ), 4,84 (s, Is, Hₐₙₒ), 4,33-3,26 (m, Is), 1,68 (s, Is, CH₂).

### Exemple 16 - synthèse de pent-4-ynyl-α-D-glucopyranoside

On synthétise de la manière indiquée ci-dessous le produit de formule (VIII) :

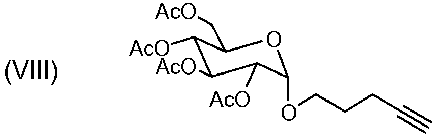

Du D-(+)-glucose (360,3 mg, 2,0 mmoles) est dissous dans du 4-pentyn-1-ol (1,01 g, 12,0 mmoles, 6,0 eq.) et la solution est chauffée à 65°C. 120 mg de H₂SO₄-SiO₂ sont ajoutés et la solution est mélangée à 65°C pendant 10 heures. La solution est refroidie à température ambiante puis le composé est purifié par chromatographie sur gel de silice avec comme mélange éluant un gradient de CH₂Cl₂ à CH₂Cl₂-MeOH ; 9:1. Le produit désiré est obtenu sous forme d'huile incolore avec un rendement de 79 % et un ratio anomérique α/β de 1,6/1. Le résidu (405,0 mg, 1,63 mmole) est dissous dans la pyridine (5 mL) et l'anhydride acétique (1,33 g, 13,0 mmoles, 8 eq.) à température ambiante.

De la DMAP (20 mg) est ensuite ajoutée à 0°C et la réaction est agitée à température ambiante pendant 5 heures. Le milieu réactionnel est neutralisé par l'addition goutte à goutte à 0°C de 5 mL de MeOH. Les solvants sont éliminés sous pression réduite à l'évaporateur rotatif et les solvants résiduels sont coévaporés 3 fois avec du toluène. La purification par chromatographie sur gel de silice avec le mélange éluant pentane-AcOEt (8/2) permet d'obtenir 190 mg de cristaux blancs du produit désiré tetraacétylé de conformation α ainsi que 450 mg du mélange des deux anomères, correspondant à un rendement total de 98 % :
RMN-¹H (D₂O, 300 MHz) : δ (ppm) : 5,47 (dd, 1H, *J* = 10,0, 9,6 Hz), 5,06 (m, 2H), 4,87 (dd, 1H, *J* = 10,2 , 3,7 Hz), 4,27 (dd, 1H, *J* = 12,3 , 4,3 Hz), 4,07 (m, 2H), 3,85 (dt, 1H, *J* = 9,9 , 5,9 Hz), 3,54 (dt, 1H, *J* = 9,9 , 6,1 Hz), 2,34 (dt, 2H, 1H, *J* = 6,8 , 2,7 Hz), 2,10 (s, 3H), 2,07 (s, 3H), 2,03 (s, 3H), 2,02 (s, 3H), 1,97 (t, 1H, J = 2,6 Hz), 1,82 (q, 2H, *J =* 6,5 Hz).
RMN-¹³C (D₂O, 75 MHz) : δ (ppm) : 170,9, 170,4, 170,3, 169,8, 96,1, 83,3, 71,0, 70,4, 69,4, 68,9, 68,7, 67,4, 66,9, 62,1, 28,4, 20,9, 20,9, 20,8, 20,8, 15,2. HRMS calculé pour C₁₉H₂₆O₁₀ [M+Na]⁺ : 437,5 ; trouvé m/z : 437,1.

### Exemple 17 - synthèse de pent-4-ynyl-α-D-glucopyranoside

On synthétise de la manière indiquée ci-dessous le produit de formule (IX) :

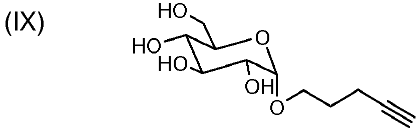

A une solution de composé tétraacétylé obtenu à l'exemple 16 (180 mg, 0,434 mmol,) dissous dans 3 mL de MeOH, est ajoutée à 0°C, 4mL de NaOMe à 0,1 M dans MeOH et le milieu réactionnel est mélangé à température ambiante pendant 4 heures. La réaction est ensuite neutralisée à pH = 7 avec une résine Dowex-50W. Le milieu réactionnel est ensuite filtré, évaporé à sec sous pression réduite et purifié par colonne chromatographie sur gel de silice avec CH₂Cl₂-MeOH ; 9:1 comme éluant pour donner le dérivé de l'a-glucose avec un rendement quantitatif sous forme d'huile incolore.
RMN-¹H (D₂O, 300 MHz) : δ (ppm) : 5,12 (d, 1H, *J* = 3,9 Hz), 4,08 (m, 2H), 4,00 (m, 1H), 3,92 (m, 2H), 3,82 (m, 1H), 3,76 (m, 1H), 3,62 (m, 1H), 2,55 (m, 3H), 2,03 (m, 2H). RMN-¹³C (D₂O, 75 MHz) : δ (ppm) : 100,0, 87,0, 75,1, 73,6, 73,3, 71,6, 71,4, 68,2, 62,4, 29,3, 16,5. HRMS calculé pour C₁₁H₁₈O₆ [M+Na]⁺ : 269,1 ; trouvé m/z : 269,0

### Exemple 18 - préparation du polymère PsAcF2 (1,2 ; 0,4)

Le polymère **PsAcF1 (1,2 ; 0,4)** (24,8 mg) et du 4-pentyn-1-ol (200 µL d'une solution à 92,5 mg/mL dans l'eau, 0,22 mmole, 2,2 eq.) sont agités dans 150 µL d'eau déionisée à température ambiante. De l'ascorbate de sodium (50 µL d'une solution fraîchement préparée dans l'eau à 200 mg/mL, 0,05 mmole, 0,5 eq.), et du sulfate de cuivre (II)-pentahydraté (50 µL d'une solution à 25 mg/mL dans l'eau, 0,005 mmole, 0,05 eq.) sont ensuite additionnés au milieu réactionnel qui est mélangé à température ambiante pendant 20 heures. La purification par dialyse dans une solution de NaCl à 2 M pendant 3 jours, puis dans de l'eau déionisée pendant 3 jours suivie de la lyophilisation conduit à l'obtention du polymère **PsAcF2 (1,2 ; 0,4)** sous forme de poudre blanche amorphe. Le degré de substitution en triazole est de 0,4 (c'est-à-dire qu'essentiellement la totalité des groupements réactifs F ont été convertis).
RMN-¹H (D₂O, 300 MHz) : δ (ppm) : 7,65 (s, Is, H_{triazole}), 5,03 (s, Is, Hₐₙₒ), 4,85 (s, Is, Hₐₙₒ), 4,30-3,30 (m, Is), 3,13 (s, Is, CH₂), 2,61 (s, Is, CH₂), 2,01 (s, Is, CH₂), 1,75 (s, Is, CH₂).

### Exemple 19 - préparation du polymère PsAcF3 (1,2 ; 0,34)

De l'ascorbate de sodium (50 µL d'une solution fraîchement préparée dans l'eau à 200 mg/mL, 0,05 mmole, 0,5 eq.), puis du sulfate de cuivre (II)-pentahydraté (50 µL d'une solution à 25 mg/mL dans l'eau, 0,005 mmole, 0,05 eq.) sont successivement ajoutés à un mélange de **PsAcF1 (1,2 ; 0,34)** (24,8 mg) et de pent-4-ynyl-α-D-glucopyranoside (49,3 mg, 0,2 mmole, 2,0 eq.) dans 900 µL d'eau déionisée à température ambiante. Après 20 heures, le milieu réactionnel est purifié par dialyse dans une solution de NaCl à 2 M pendant 3 jours, puis dans de l'eau déionisée pendant 2 jours. Le polymère **PsAcF3 (1,2 ; 0,34)** est obtenu après lyophilisation sous forme d'une poudre blanche amorphe. Le degré de substitution en triazole est de 0,26, c'est-à-dire que seulement une partie des groupements réactifs F ont été convertis.
RMN-¹H (D₂O, 300 MHz) : δ (ppm) : 7,74 (s, Is, H_{triazole}), 5,06 (s, Is, Hₐₙₒ), 4,88 (s, Is, Hₐₙₒ), 4,78 (s, Is, Hₐₙₒ), 4,34-3,16 (m, Is), 2,71 (s, Is, CH₂), 2,05 (s, Is, CH₂), 1,89 (s, Is, CH₂).

### Exemple 20 - préparation du polymère PsAcArF3 (1,2 : 0,42 : 0,38)

De l'ascorbate de sodium (50 µL d'une solution fraîchement préparée dans l'eau à 200 mg/mL, 0,05 mmole, 0,5 eq.), puis du sulfate de cuivre (II)-pentahydraté (50 µL d'une solution à 25 mg/mL dans l'eau, 0,005 mmole, 0,05 eq.) sont successivement ajoutés à un mélange de **PsAcArF2 (1,2 ; 0,42, ; 0,38)** (28,7 mg) et de 4-pentyn-1-ol (200 µL d'une solution à 92,5 mg/mL dans l'eau, 0,22 mmole, 2,2 eq.) dans 700 µL d'eau déionisée à température ambiante. Après 20 heures, le milieu réactionnel est purifié par dialyse dans une solution de NaCl à 2 M pendant 3 jours, puis dans de l'eau déionisée pendant 2 jours. Le polymère **PsAcArF3 (1,2 ; 0,42 ; 0,38)** est obtenu après lyophilisation sous forme d'une poudre blanche amorphe. Le degré de substitution en triazole est de 0,28 (taux de conversion de 74 % des groupements réactifs F).
¹H RMN (300 MHz, D₂O) δ (ppm) : 7,62 (s, Is, H_{triazole}), 7,21 (s, Is, Hₐᵣ), 5,04 (s, Is, Hₐₙₒ), 4,89 (s, Is, Hₐₙₒ), 4,29-3,50 (m, Is), 3,13 (s, Is, CH₂), 2,58 (s, Is, CH₂), 1,96 (s, Is, CH₂), 1,73 (s, Is, CH₂).

### Exemple 21 - préparation du polymère PsAcArF4 (1,2 ; 0,42 ; 0,38)

A une solution de **PsAcArF2 (1,2 ; 0,42 ; 0,38)** (28,7 mg) et de pent-4-ynyl-α-D-glucopyranoside (43,8 mg, 0,178 mmole, 1,8 eq.) dans 500 µL d'eau déionisée et 400 µL de PBS à 0,1 M est ajoutée de l'ascorbate de sodium (100µL à 200 mg/mL dans le PBS, 0,05 mmole, 1 eq.). Du sulfate de cuivre (II) pentahydraté (100 µL de 25 mg/mL dans le PBS, 0,005 mmole, 0,1 eq.) est ajouté et le milieu réactionnel est agité à température ambiante pendant 20 h. Le dextran est repris dans une solution aqueuse d'EDTA-2Na à 0,5 M (2 mL) avant d'être purifié par dialyse dans une solution de NaCl à 2 M pendant 3 jours, puis dans de l'eau déionisée pendant 2 jours. La lyophilisation permet l'obtention du **PsAcArF4 (1,2 ; 0,42 ; 0,38)** sous forme d'une poudre amorphe blanche. Le degré de substitution en triazole est de 0,34 (taux de conversion de 89 % des groupements réactifs F).
¹H RMN (300 MHz, D₂O) δ (ppm) : 7,63 (s, Is, H_{triazole}), 7,21 (s, Is, Hₐᵣ), 5,00 (s, Is, Hₐₙₒ), 4,90 (s, Is, Hₐₙₒ), 4,85 (s, Is, Hₐₙₒ), 4,23-3,22 (m, Is), 3,09 (s, Is, CH₂), 2,64 (s, Is, CH₂), 1,22 (s, Is, CH₂), 1,19 (s, Is, CH₂).

### Exemple 22 - fonctionnalisation de plaques de polystyrène et mesure de l'angle de goutte

Une plaque de polystyrène (plaque Goodfellow, 10 cm × 10 cm) préalablement lavée à l'eau et à l'éthanol, est trempée de moitié dans une solution de polymère dans le tampon PBS à une concentration de 10 µg/mL ou dans le PBS seul (témoin) pendant une nuit à température ambiante (une plaque par type de polymère). Les polymères testés sont ceux décrits ci-dessus et plus particulièrement le **PsAc1 (1,2),** le **PsAcAr1 (1,2 ; 0,42),** le **PsAcArF2 (1,2 ; 0,42 ; 0,38)** et le **PsAcArF4 (1,2 ; 0,42 ; 0,27)** (la valeur de 0,27 représentant ici uniquement le degré de substitution en triazole).

La surface de polystyrène en contact avec la solution est ensuite trempée deux fois 5 minutes dans un bain d'eau. Elle est séchée sous azote pendant 10 min.

Ces surfaces sont ensuite utilisées pour les mesures d'angle de goutte. La zone du substrat n'ayant pas été en contact avec la solution est utilisée comme témoin.

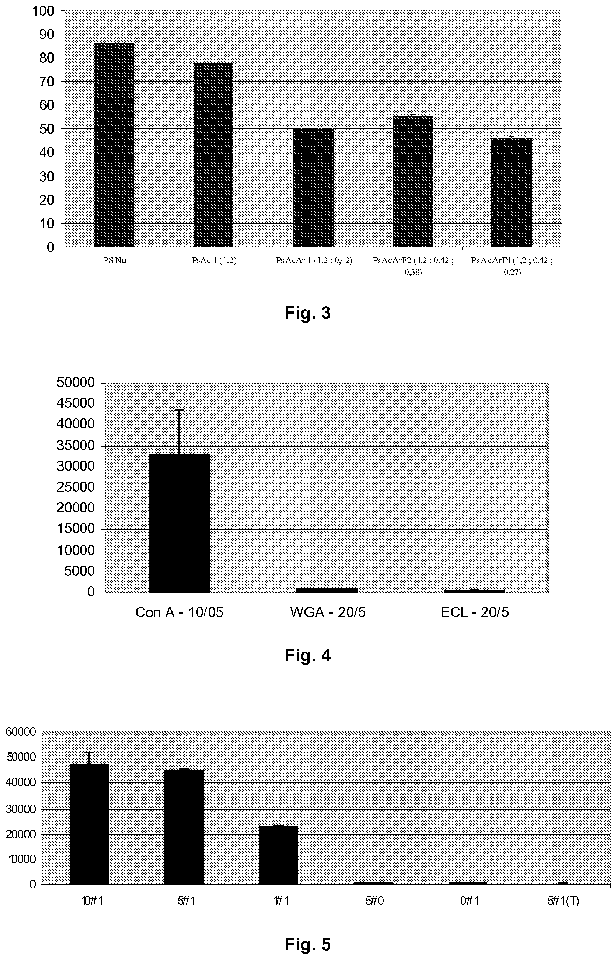

10 gouttes d'eau de 1 µL sont déposées au milieu de la surface traitée et non traitée (témoin). Les résultats (médiane de l'angle et intervalle interquartile) sont représentés sur la **figure 3****.**

On constate que le traitement de la surface polystyrène par les polysaccharides **PsAcAr1, PsAcArF2** et **PsAcArF4** conduit à une diminution significative de l'angle de goutte de l'eau comparativement au polystyrène non traité. Ces polymères permettent donc une fonctionnalisation du polystyrène.

### Exemple 23 - fonctionnalisation de plaques à 96 puits en polystyrène par des ligands

Dans cet exemple, le fond des puits en polystyrène est entièrement recouvert d'une couche de PsAc, PsAcAr ou PsAcArF puis incubé avec des ligands (lectines). Une protéine est ensuite incubée avec la surface fonctionnalisée par les ligands. Enfin, un système de détection par fluorescence permet d'identifier les puits ayant capturé la protéine cible.

Les plaques 96 puits utilisées sont de type MaxiSorp Immunoplate du manufacturier Nunc.
1) 100 µL d'une solution de polymère dans du PBS (pH=7,4) (entre 50 µg/mL et 0,1 µg/mL) sont incubés dans les puits de la plaque pendant 16 h à température ambiante.
2) Les puits sont ensuite lavés avec 3×300 µL de PBS (pH=7,4) contenant 0,1 % de Tween 20 pendant 3×5 minutes.
3) Les puits sont ensuite incubés avec 300 µL d'une solution de PBS (pH=7,4) Tween 20 (0,1%) contenant de la BSA (3 %, 1 % ou 0,1 %) ou de la caséine (0,1%) pendant 1 heure à température ambiante.
4) Les puits sont lavés avec 3×300 µL de PBS (pH=7,4) Tween 20 (0,1 %) pendant 3×5 minutes.
5) Les puits sont ensuite incubés avec 200 µL d'une solution de lectine biotinylée (par exemple 10 µg/mL, 5 µg/mL ou 1 µg/mL) dans du PBS (pH=7,4) Tween 20 (0,1%) contenant MnCl₂ et CaCl₂ à une concentration finale de 1 mM. L'incubation dure 1 heure à température ambiante sous agitation.
   Les lectines suivantes (disponibles commercialement) ont été utilisées : concanavaline A (ConA), agglutinine de germe de blé (WGA) et lectine d'*Erythrina Cristagalli.*
   La ConA interagit avec des résidus de alpha-D-glucose ou alpha-D-mannose terminaux. La WGA reconnait les N-acétyl-D-glucosamines. La ECL reconnait les résidus d'alpha-D-galactose terminaux.
6) Les puits sont lavés avec 3×300 µL de PBS (pH=7,4) Tween 20 (0,1 %) pendant 3×5 minutes.
7) Les puits sont ensuite incubés avec 200 µL d'une solution de streptavidine marquée à la tétraméthylrhodamine (5 µg/mL ou 1 µg/mL) dans du PBS (pH=7,4) Tween 20 (0,1%) pendant 2 heures à température ambiante.
8) Les puits sont lavés avec 3×300 µL de PBS (pH=7,4) Tween 20 (0,1 %) pendant 3×5 minutes.
9) Les puits sont lavés avec 3×300 µL d'eau déionisée pendant 3×5 minutes.
10) La plaque est séchée à la centrifugeuse pendant 10 min puis séchée à l'azote pendant 5 min.
11) La fluorescence au fond des puits est mesurée à 532 nm à l'aide d'un scanner de fluorescence confocal.

Les résultats sont représentés dans les **figures 4** et **5**.

La **figure 4** est un histogramme représentant l'intensité de fluorescence obtenue avec le polymère **PsAcArF4 (1,2 ; 0,42, 0,34)** (le taux de substitution de 0,34 visant uniquement les groupements F triazoles) et les trois lectines susmentionnés. Les rapports indiqués sur l'axe des abscisses sont les rapports lectine / streptavidine.

On constate que le **PsAcArF4** est reconnu sélectivement par la ConA comme attendu, montrant la qualité de la fonctionnalisation de la plaque de polystyrène et la disponibilité du ligand vis-à-vis de la protéine cible.

La **figure 5** est un histogramme représentant l'intensité de fluorescence obtenue avec le même polymère **PsAcArF4 (1,2 ; 0,42, 0,34)** et la ConA. Les rapports indiqués sur l'axe des abscisses sont les rapports lectine / streptavidine. Le dernier résultat (T) est un témoin sans le polysaccharide.

Cette expérience illustre la relation entre l'intensité du signal de fluorescence et la concentration de ConA utilisée dans les puits. La fluorescence associée aux puits n'ayant pas reçu de ConA, de streptavidine ou de **PsAcArF4** est très faible.

### Exemple 24 - formation de microplots au fond des puits d'une plaque à 96 puits par impression de polysaccharides, interaction avec les lectines

Dans cette expérience, différents polymères sont imprimés dans le fond d'une plaque à 96 puits non traitée.

Les biopuces ainsi réalisées sont ensuite incubées avec des protéines (lectines).

Les plaques à 96 puits utilisées sont de types MaxiSorp Immunoplate du manufacturier Nunc. 300 pL de solution de polymère (aux concentrations de 10 µg/mL, 7,5 µg/mL, 5 µg/mL et 1 µg/mL dans du PBS) sont déposés sur la plaque au moyen d'un appareil Packard BioChipArrayer BCA-1 suivant la disposition suivante :

| | | | |
|---|---|---|---|
| PsAcArF4 (1,2 ; 0,42 ; 0,27) | ○○○ | ○○○ | PsAc1 (1,2) |
| PsAcF1 (1,2 ; 0,38) | ○○○ | ○○○ | PsAcAr1 (1,2 ; 0,42) |
| PsAcArF2 (1,2 ; 0,42 ; 0,38) | ○○○ | ○○○ | PsAcArF3 (1,2 ; 0,42 ; 0,28) |

Pour le polymère **PsAcArF4,** le taux de substitution de 0,27 correspond aux groupements triazoles uniquement. Pour le polymère **PsAcArF3** le taux de substitution de 0,28 correspond aux groupements triazoles uniquement.
1) Les puits sont ensuite incubés avec 100 µL d'une solution de PBS (pH=7,4) Tween 20 (0,1%) contenant de la BSA (3 %) pendant 15 minutes à température ambiante.
2) Les puits sont ensuite lavés avec 3×100 µL de PBS (pH=7,4) contenant 0,1 % de Tween 20 pendant 3×5 minutes.
3) Les puits sont ensuite incubés avec 100 µL d'une solution de lectine biotinylée (par exemple 20 µg/mL, 10 µg/mL ou 5 µg/mL) dans du PBS (pH=7,4) Tween 20 (0,1%) pendant 1 heure à température ambiante au sous agitation orbitalaire. Pour les lectines (ConA, ECL et WGA), des sels de MnCl₂ et CaCl₂ sont ajoutés pour une concentration finale de 1 mM.
4) Les puits sont lavés avec 3×100 µL de PBS (pH=7,4) Tween 20 (0,1 %) pendant 3×5 minutes.
5) Les puits sont ensuite incubés avec 100 µL d'une solution de streptavidine tétraméthylrhodamine (5 µg/mL) dans du PBS (pH=7,4) Tween 20 (0,1%) pendant 2 heures à température ambiante.
6) Les puits sont lavés avec 3×100 µL de PBS (pH=7,4) Tween 20 (0,1 %) pendant 3×5 minutes.
7) Les puits sont lavés avec 3×100 µL d'eau déionisée pendant 3×5 minutes.
8) La plaque est séchée à la centrifugeuse pendant 10 min puis séchée à l'azote pendant 5 min.
9) La détection est réalisée au scanner à 532 nm.

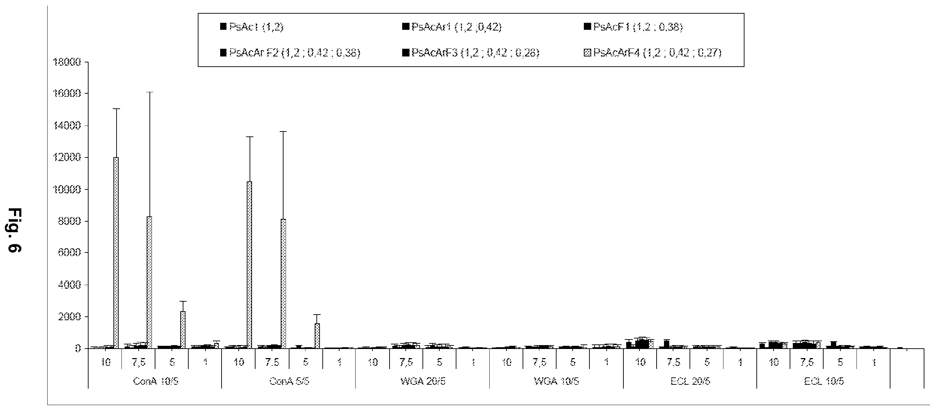

La **figure 6** résume les résultats obtenus. On constate que la ConA se fixe sélectivement sur le polymère **PsAcArF4,** aucune des deux autres lectines n'étant fixées.

### Exemple 25 - fonctionnalisation de plaques de polystyrène et mesure de l'angle de goutte, influence du taux de substitution aromatique

Pour cette expérience on utilise les polymères **PsAc1 (1,27), PsAcAr1 (1,27** ; **0,03), PsAcAr1 (1,27 ; 0,39)** et **PsAcAr1 (1,27 ; 0,54).**

5 plaques de polystyrène (plaques Goodfellow, 10 cm × 10 cm) préalablement lavées à l'eau et à l'éthanol, sont trempées de moitié dans les solutions de **PsAc1** ou **PsAcAr1** solubilisés dans un tampon PBS à une concentration de 10 µg/mL ou dans du PBS seul pendant une nuit à température ambiante.

La surface de polystyrène en contact avec la solution est alors trempée deux fois 5 minutes dans un bain d'eau. Elle est ensuite séchée sous azote pendant 10 min.

Ces surfaces sont ensuite utilisées pour les mesures d'angle de goutte. La zone du substrat n'ayant pas été en contact avec la solution est utilisée comme témoin.

10 gouttes d'eau de 1 µL sont déposées au milieu de la surface traitée et non traitée (témoin).

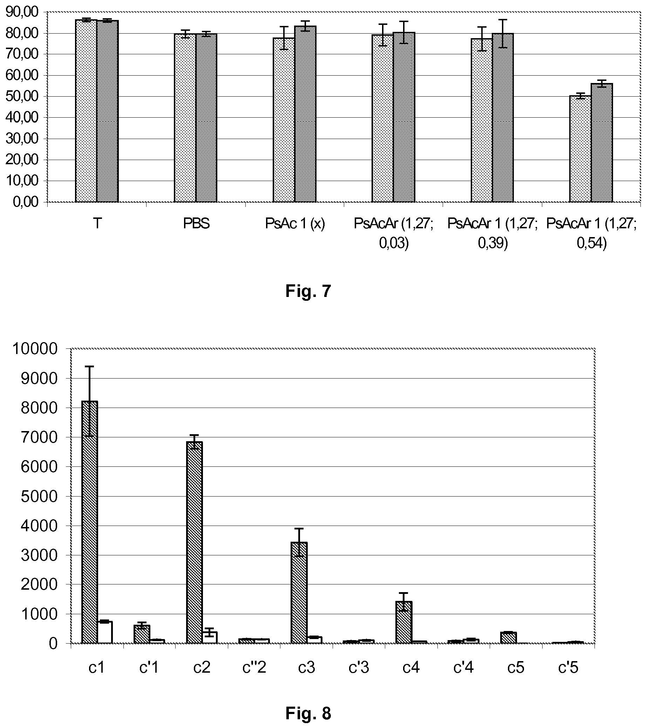

Les résultats (médiane de l'angle et intervalle interquartile) sont représentés sur la **figure 7****.** La première barre est le résultat de mesure immédiat, et la deuxième barre après 24 h de stockage sous vide partiel (dessiccateur).

On constate que le **PsAcAr1 (1,27 ; 0,54)** permet une baisse significative de l'angle de contact, qui passe de 86°C pour le polystyrène non traité à 56°C pour le polystyrène traité. Les fonctions greffées à la surface du polystyrène à l'aide de **PsAcAr1 (1,27 ; 0,54)** rendent la surface plus hydrophile, ce qui se traduit par une diminution de l'angle de contact de l'eau sur ces surfaces, par rapport au polystyrène non traité. Le polymère **PsAcAr1 (1,27 ; 0,54)** permet ainsi la fonctionnalisation chimique du polystyrène.

### Exemple 26 - fonctionnalisation de plaques de 96 puits en polystyrène et ligation hydrazone avec des peptides fluorescents

Pour cette expérience, on utilise les polymères **PsAcArF1 (1,1 ; 0,75 ; 0,03)** et **PsAcAr 1 (1,1 ; 0,75).** Le polymère **PsAcArF 1 (1,1 ; 0,75 ; 0,03)** permet de fonctionnaliser le fond des puits par une fonction hydrazide. Le polymère **PsAcAr1 (1,1 ; 0,75),** qui ne porte pas cette fonction, sert de témoin. La fonction hydrazide est connue pour réagir avec les molécules portant une fonction aldéhyde. Le lien formé est une hydrazone. Le principe de la réaction est le suivant :

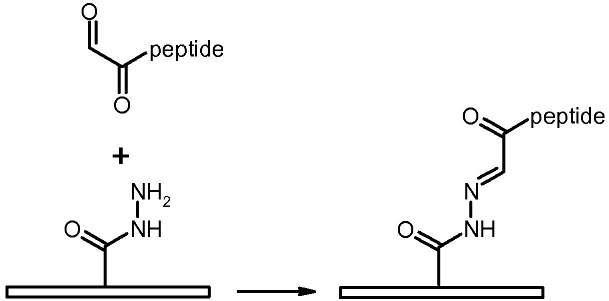

Deux peptides sont utilisés : le peptide 1 (SEQ ID NO : 1) de formule Rho-KR-NH(CH₂)₃-NH-CHOCO (fonctionnalisé par la tétraméthylrhodamine et par une fonction CHOCO) et le peptide 2 (SEQ ID NO : 2) de formule Rho-KR-NH₂ (fonctionnalisé par la tétraméthylrhodamine et par une fonction amide). La synthèse de ces peptides est décrite dans Ollivier et al., Alpha-oxo semicarbazone peptide or oligodeoxynucleotide microarrays, Bioconjug. Chem. 14, 430-9 (2003).

### Protocole :

Les puits d'une microplaque à 96 puits en polystyrène (Maxisorp, Nunc) sont traités avec 100 µL d'une solution de **PsAcArF1 (1,1** ; **0,75 ; 0,03)** ou de **PsAcAr1 (1,1 ; 0,75)** à une concentration de 5 µg/mL dans le PBS. Le traitement est réalisé à température ambiante sous agitation pendant une nuit.

La plaque est ensuite lavée au PBS / Tween 20 0,05% à l'aide d'un laveur de plaques (300 µL/puits, 6 lavages).

Deux séries de solutions de peptides 1 et 2 sont préparées à des concentrations de 10⁻⁶, 5×10⁻⁷, 2,5×10⁻⁷, 1,25×10⁻⁷ et 6,25×10⁻⁸ M dans un tampon acétate pH 5,5 / 0,1% de BSA. 60 µL de chaque solution est déposé au fond des puits traités par **PsAcArF1 (1,1** ; **0,75 ; 0,03)** ou **PsAcAr1 (1,1 ; 0,75)** (2 puits / condition). La réaction de ligation s'effectue sous agitation pendant 2h à température ambiante.

La plaque est lavée au PBS / Tween 20 (0,05 %) (300 µL/puits, 6 lavages) puis à l'eau déionisée (300µL/puits, 3 lavages). Un séchage à la centrifugeuse est alors réalisé (2500 tours/min, 5min, 20°C). La lecture de la plaque s'effectue au scanner de fluorescence Técan.

Le résultat est représenté sur la **figure 8**. Les barres grises correspondent au peptide 1 et les barres blanches au peptide 2. Cn correspond aux différentes concentrations de **PsAcArF1 (1,1 ; 0,75 ; 0,03)** et C'n correspond aux différentes concentrations de **PsAcAr1 (1,1 ; 0,75)** avec 1 = 10⁻⁶ M ; 2 = 5×10⁻⁷ M ; 3 = 2,5×10⁻⁷ M ; 4 = 1,25×10⁻⁷ M ; 5 = 6,25.10⁻⁸ M.

L'intensité des signaux (en ordonnée) qui sont associés aux puits traités par **PsAcArF1 (1,1 ; 0,75 ; 0,03)** puis incubés avec le peptide aldéhyde 1 est élevée (Cn, peptide 1). Comparativement, l'intensité des signaux associés aux puits traités par le **PsAcAr1 (1,1 ; 0,75)** (puits témoin qui ne possèdent pas la fonction hydrazide réactive vis-à-vis de la fonction aldéhyde du peptide 1) est très faible (C'n, peptide 1).

Dans tous les cas, l'intensité des signaux associés aux puits incubés avec le peptide contrôle 2 ne possédant pas la fonction aldéhyde est très faible (Cn ou C'n, peptide 2).

Cet exemple démontre la liaison chimiosélective du peptide aldéhyde 1 aux puits traités par **PsAcArF1 (1,1 ; 0,75; 0,03)** et qui présentent des fonctions hydrazides accessibles.

Des expériences complémentaires ont été effectuées en faisant varier le pH de la réaction de ligation. Le pH préféré pour la réaction est de 5 comme attendu pour ce type de réaction chimique.

### Exemple 27 - fonctionnalisation de plaques de 96 puits en polystyrène, préparation de biopuces par impression de peptides et réaction in situ

Dans cette expérience, les puits sont fonctionnalisés par un polymère polysaccharide. Des peptides sont imprimés pour former une biopuce, les peptides se liant à la surface par la formation d'un lien covalent hydrazone.

La synthèse des peptides utilisés dans cette expérience a été décrite dans :
Carion et al., Chemical Micropatterning of Polycarbonate for Site-Specific Peptide Immobilization and Biomolecular Interactions. Chembiochem 8, 315-322 (2007).

Les peptides utilisés sont :
- Ser-HA : H-SGYPYDVPDYAGYPYDVPDYAGYPYDVPDYAS-NH₂ (SEQ ID NO : 3) ;
- Ser-FLAG : H-SDYKDHDGDYKDHDIDYKDDDDKGGS-NH₂ (SEQ ID NO : 4) ;
- CHOCO-HA : CHOCO-GYPYDVPDYAGYPYDVPDYAGYPYDVPDYASNH₂ (SEQ ID NO : 5) ;
- CHOCO-FLAG : CHOCO-DYKDHDGDYKDHDIDYKDDDDKGGS-NH₂ (SEQ ID NO : 6).

### Protocole :

Impression des peptides Ser-HA, CHOCO-HA, Ser-FLAG et CHOCO-FLAG, à une concentration de 10⁻⁴ M dans du tampon PBS ou du tampon Acétate à pH 5,5, sur des plaques de type 96 puits fonctionnalisées avec le polymère **PsAcArF1** (10 µg/mL), le polymère **PsAcAr1** (10 µg/mL), le tampon PBS ou non traitées.

La saturation des puits est réalisée avec 300 µL de PBS+1%BSA pendant 30 min sous agitation.

Les puits sont lavés manuellement au PBS / 0,1% Tween 20.

On réalise une incubation d'anticorps anti-HA et anti-FLAG à une concentration de 1 µg/mL dans du PBS / 0,1%BSA pendant 1h30 sous agitation (100 µL/puits). Les puits sont lavés manuellement au PBS / 0,1% Tween 20.

On réalise une incubation d'anticorps anti-IgG murine marqués à la tétraméthylrhodamine à une concentration de 2 µg/mL dans PBS / 0,1%BSA pendant 1h sous agitation (100 µL/puits).

Les puits sont lavés manuellement au PBS / 0,1% Tween 20, 3 fois à l'eau, séchés par centrifugation (2500 tours/min, 5 min, 20°C).

Les plaques sont lues au scanner Técan (focus offset : -1000 / MTP gain : 90 / résolution : 4µm).

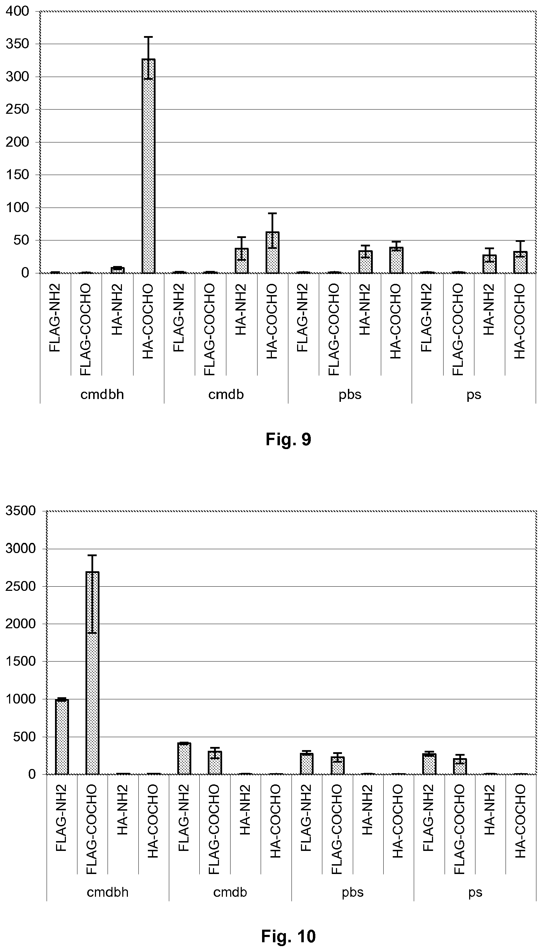

Les résultats de l'incubation avec l'anticorps anti-HA sont représentés à la **figure 9** et les résultats de l'incubation avec l'anticorps anti-FLAG sont représentés à la **figure 10** (intensité de fluorescence en ordonnée).

### Exemple 28 - préparation de la bis({2-[triphénylméthyl)sulfanyl]éthyl})amine

1,50 g de bis(2-chloroéthyl)amine (8,4 mmol) et 4,65 g de triphénylméthanethiol (2 équivalents, 16,80 mmoles) sont introduits dans un ballon et placés sous atmosphère inerte. Sous agitation magnétique, 25 mL de diméthylformamide (DMF) anhydre sont ajoutés et le mélange réactionnel est refroidi dans un bain de glace. 4 équivalents de 1,8-diazabicyclo[5,4,0]undec-7-ène (DBU) sont additionnés goutte à goutte au mélange. Le mélange est laissé sous agitation à température ambiante durant 3 heures et la réaction est suivie par chromatographie sur couche mince (CCM) (éluant: cyclohexane / acétate d'éthyle / triéthylamine: 8 / 2 / 0,1). Passé ce délai, le solvant est évaporé à l'évaporateur rotatif. Le solide blanc obtenu est alors dissous dans 50 mL de dichlorométhane (DCM) et le produit est extrait trois fois avec une solution aqueuse de KH₂PO₄ à 5 %. Le produit est alors purifié par chromatographie sur colonne de gel de silice (éluant : cyclohexane / AcOEt / triéthylamine (TEA) : 8 / 2 / 0,1) pour obtenir 1,46 g de solide amorphe blanc (rendement de 28 %).

L'analyse du produit est la suivante.
Rf = 0,37 (silica gel, cyclohexane / AcOEt / TEA : 8 / 2 / 0,1).
¹H RMN (300 MHz, CDCl₃) δ 7,41 -7,37 (m, 12H, Trt), 7,15 - 7,29 (m, 18H, Trt), 2,23 - 2,36 (m, 8H, CH₂), 1,26 (s, 1H, NH).
¹³C (75 MHz, CDCl₃) 154,1 ; 129,8 ; 128,1 ; 126,9 ; 47,9 ; 32,6 ; MALDI-TOF: 243,1 [Trt⁺], 622,3 [M+H⁺]⁺, 644,3 [M⁺+Na⁺].

### Exemple 29 - synthèse du Boc- βAla-N(CH₂CH₂STrt)₂

On dissout de la Boc-β alanine (1,908 g, 10,1 mmol) dans 50 ml de dichlorométhane, puis la solution est refroidie à 0°C et on ajoute du N,N-dicyclohexyl-carbodiimide (5 mmol). L'agitation est maintenue pendant 30 minutes à température ambiante. Après filtration, on ajoute de la bis({2-(triphénylméthyl)sulfanyl]éthyl})amine (2,5 g, 4 mmol). Le mélange réactionnel est laissé sous agitation pendant 2 heures à température ambiante. On évapore à sec le milieu réactionnel. Au résidu obtenu, on additionne 100 mL d'une solution aqueuse de soude (1 N) et on extrait avec du dichlorométhane (3 x 100 mL). La phase organique est séchée sur sulfate de magnésium anhydre, et évaporée. Le produit brut est purifié par chromatographie sur gel de silice (cyclohexane / acétate d'éthyle / triéthylamine, 80/20/0,1, v/v/v). On obtient le produit Boc-βAla-N(CH₂CH₂STrt)₂ sous forme d'une poudre blanche (3,1 g, 3,9 mmol, 97%).

Le schéma de la réaction est le suivant :

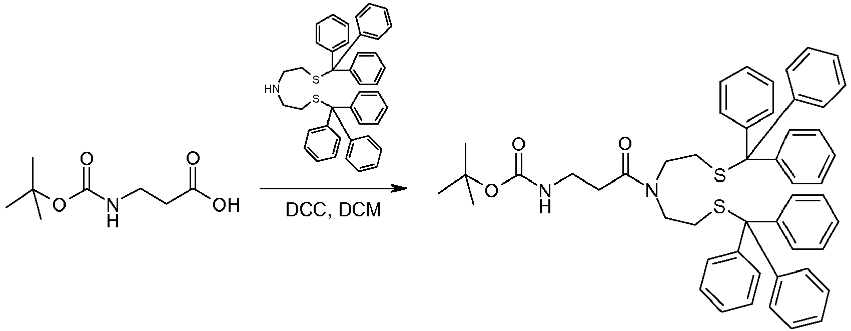

Point de fusion : 53°C.
RMN-¹H (300 MHz, CDCl₃) : *δ* ppm 1,43 (9 H, s, CH₃) ; 1,94 (2 H, t, J = 4 Hz, CH₂S) ; 2,14 (2 H, t, J = 4 Hz, CH₂S) ; 2,31 (2 H, t, J = 4 Hz, CH₂CON) ; 2,73 (4 H, m, CONCH₂) ; 3,23 (2 H, m, OCONCH₂) ; 5,23 (H, m, CONH) ; 7,10-7,50 (30 H_{Ar}, m). RMN-¹³C (75 MHz, CDCl₃) : *δ* ppm 28,50 (CH₃) ; 29,50 (CH₂) ; 30,0 (CH₂) ; 33,0 (CH₂) ; 36,0 (CH₂) ; 45,0 (CH₂) ; 47,0 (CH₂) ; 126,7 (CH) ; 127,0 (CH) ; 128,0 (CH) ; 129,5 (CH) ; 130,0 (CH) ; 143,0 (Cquat) ; 144,5 (Cquat) ; 145,0 (Cquat) ; 156,0 (Cquat) ; 171,0 (Cquat).
MALDI-TOF (matrice DHB) : Mcalc = 792,3 (C₅₀H₅₂N₂O₃S₂) ; m/z = 815,4 [M + Na]⁺; m/z = 831,3 [M+K]⁺.

### Exemple 30 - synthèse du Boc-βAla-dithiazépane

Le produit le produit Boc-βAla-dithiazépane (3 g, 3.,78 mmol) est dissous dans le dichlorométhane (50 ml), et on ajoute de l'iode (2,87 g, 11,3 mmol). L'agitation est maintenue pendant 30 minutes à température ambiante. L'excès d'iode est neutralisé par une solution aqueuse de thiosulphate de sodium (3 M, 100 ml). On extrait avec du dichlorométhane (4×100 mL). La phase organique est séchée sur sulfate de magnésium anhydre, et évaporée. Le produit brut est purifié par chromatographie sur gel de silice (hexane / acétate d'éthyle / triéthylamine, 40 / 60 / 0,1, v/v/v). Le produit Boc-βAla- dithiazépane est obtenu sous forme d'une patte blanche (1,1 g, 3,6 mmol, 95%).

Le schéma de la réaction est le suivant :

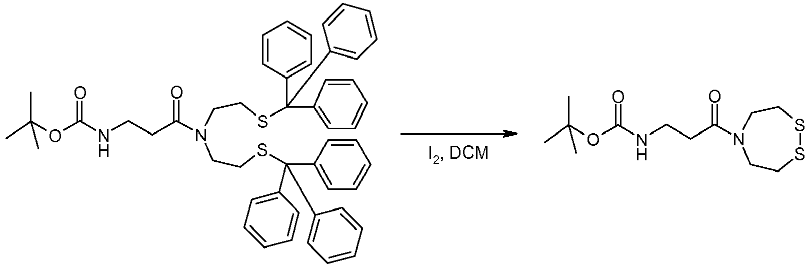

RMN-¹H (300 MHz, CDCl₃) : *δ* ppm 1,39 (9 H, s, CH₃) ; 2,48 (2 H, t, J = 4 Hz, CH₂CON) ; 2,87 (2 H, t, J = 4 Hz, CH₂S) ; 3,04 (2 H, t, J = 4 Hz, CH₂S) ; 3,39 (2 H, m, OCONCH₂) ; 3,76 (2 H, t, J = 4 Hz, CONCH₂) ; 3,86 (2 H, t, J = 4 Hz, CONCH₂ ; 5,25 (H, m, NH).
RMN-¹³C (75 MHz, CDCl₃) : *δ* ppm 28,5 (CH₃) ; 33,0 (CH₂) ; 36,0 (CH₂) ; 37,0 (CH₂) ; 39,5 (CH₂) ; 50,0 (CH₂) ; 53,0 (CH₂) ; 143,0 (Cquat) ; 156,0 (Cquat) ; 172,0 (Cquat). MALDI-TOF (matrice DHB) : Mcalc = 306,1 (C₁₂H₂₂N₂OS₂) ; m/z = 307,1 [M + H]⁺; m/z = 329,1 [M + Na]⁺; m/z = 345,1 [M+K]⁺.

### Exemple 31 - synthèse du NH₂-βAla-dithiazépane

Le produit Boc-βAla-dithiazépane (1 g, 3,26 mmol) est dissous dans le dichlorométhane (5 ml), et on ajoute de l'acide trifluoroacétique (5 mL). L'agitation est maintenue pendant 30 minutes à température ambiante. On évapore à sec le milieu réactionnel. Au résidu obtenu, on additionne une solution aqueuse de soude (1 N) et on extrait avec du dichlorométhane (3 x 100 mL). La phase organique est séchée sur sulfate de magnésium anhydre, et évaporée. Le produit NH₂-βAla-dithiazépane est obtenu sous forme d'une huile translucide (628 mg, 3,04 mmol, 94%).

Le schéma de la réaction est le suivant :

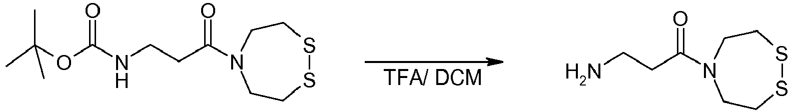

RMN-¹H (300 MHz, CDCl₃) : *δ* ppm 2,44 (2 H, t, J = 4 Hz, CH₂CON) ; 2,60 (H, m, NH₂) ; 2,90 (2 H, t, J = 4 Hz, NH₂CH₂) ; 3,0 (4 H, m, CH₂S) ; 3,77 (2 H, t, J = 4 Hz, CONCH₂) ; 3,84 (2 H, t, J = 4 Hz, CONCH₂).
RMN-¹³C (75 MHz, CDCl₃) : *δ* ppm 35,0 (CH₂) ; 35,5 (CH₂) ; 37,5 (CH₂) ; 39,0 (CH₂) ; 50,0 (CH₂) ; 52,0 (CH₂) ; 172,0 (Cquat).
MALDI-TOF (matrice DHB): Mcalc = 206,05 (C₇H₁₄N₂OS₂) ; m/z = 207,0 [M + H]⁺; m/z = 229,0 [M + Na]⁺.

### Exemple 32 - synthèse du polymère PsAcArF5

On dissout dans un ballon 50 mg de polymère **PsAcAr1 (1,1 ; 0,49)** (0,20 mmol) dans 1 mL d'eau déionisée, puis on ajuste le pH à 4,74 avec une solution de HCl à 1N. On ajoute du 1-cyclohexyl-3-(2-morpholino-ethyl)carbodiimide (CMC) (100 mg, 0,22 mmol, 1,1 eq), puis on ajuste le pH à 4,74. On ajoute l'amine H₂N(CH₂)₂CON(CH₂CH₂S-)₂ (75 mg, 0,36 mmol, 1,8 eq). Le mélange réactionnel est agité pendant 1 nuit à température ambiante.

Le milieux réactionnel est ensuite disposé dans un boudin de dialyse (3500 g/mol ; 1 mL/cm), puis plongé dans 2,5 L d'une solution de NaCl à 2M pour y rester pendant 5 jours puis dans 2,5 L d'eau pour y rester également pendant 5 jours.

Le contenu du boudin est alors mis dans un pot afin d'être lyophilisé pour donner 47 mg de **PsAcArF5.** Le schéma de la réaction est le suivant :

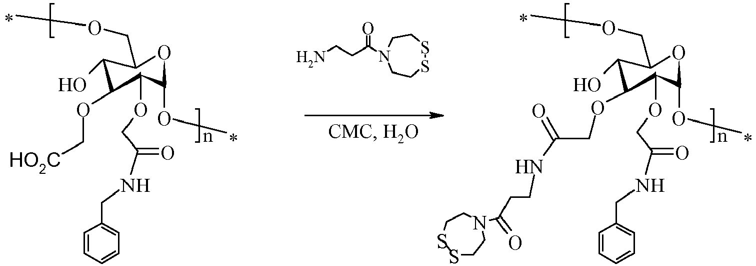

### Exemple 33 - fonctionnalisation chimique de plaques à 96 puits en polystyrène, ligation amide avec des peptides fluorescents

Dans cet exemple, les polymères **PsAcAr1** et **PsAcArF5** ont été utilisés.

Le polymère **PsAcArF5** permet de fonctionnaliser le fond des puits par une fonction CON(CH₂CH₂S-)₂. Le polymère **PsAcAr1,** qui ne porte pas cette fonction, sert de témoin. La fonction CON(CH₂CH₂S-)₂ peut réagir avec les molécules portant une fonction beta-amino thiol, comme par exemple une cystéine présente en position N-terminale de peptides. Le lien formé est une liaison amide.

La chimie d'immobilisation par le polymère **PsAcArF5** est décrite dans le schéma ci-dessous :

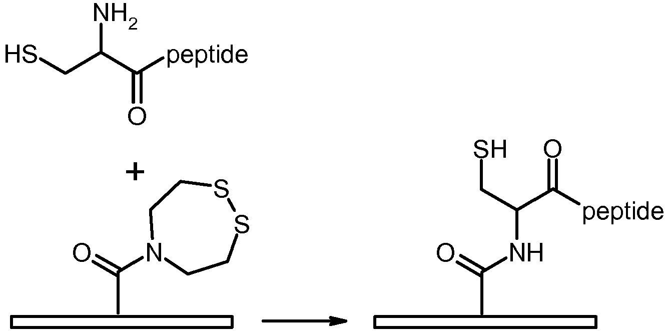

Dans le présent exemple, deux peptides sont utilisés :
1) H-CILK(rhodamine)EPVHGV-NH₂ (SEQ ID NO : 7) ;
2) H-SILK(rhodamine)EPVHGV-NH₂ (SEQ ID NO : 8).

Ces deux peptides sont synthétisés selon le protocole suivant :
Le peptide H-ILK(Mtt)E(*t*Bu)PVH(Trt)GV-NH₂ (SEQ ID NO : 9) est assemblé sur une résine Novasyn TGR® (Novabiochem, 0,25 mmol, 0,23 mmol/g) utilisant le *Microwave Peptide Synthesizer* (CEM) et la stratégie Fmoc/*tert*-butyle. La déprotection du groupe Mtt est réalisée avec une solution à 1% TFA dans CH₂Cl₂ (17 × 15 ml). La résine est neutralisée avec une solution à 5% DIEA dans CH₂Cl₂ (5 × 2 min) puis lavée avec du CH₂Cl₂ (3 × 2 min), puis du DMF (3 × 2 min). La résine est ensuite mise en réaction pendant 2 h avec la 5(6)-carboxytétraméthylrhodamine (150,6 mg, 0,35 mmol), l'HOBT (47 mg, 0,35 mmol), l'HBTU (132,7 mg, 0,35 mmol) et la DIEA (217,8 µl, 1,75 mmol, 5 éq) dans le DMF. La résine est ensuite lavée avec du DMF (4 × 2min). Le groupe terminal Fmoc est déprotégé avec une solution de pipéridine à 20 % dans le DMF (5 et 15 min). La résine est lavée avec du DMF (4 × 2 min). La résine est divisée en deux portions et mise en réaction avec Fmoc-Cys(Trt)-OH ou Fmoc-Ser(*t*Bu)-OH respectivement. Le couplage est réalisé manuellement en utilisant 4 équivalents d'acide aminé protégé, 4 équivalents d'HBTU et d'HOBT et 12 équivalents de DIEA. La résine est lavée avec du DMF (4 × 2 min). Le groupe terminal Fmoc est déprotégé avec une solution de pipéridine à 20 % dans le DMF (5 et 15 min). La résine est ensuite lavée avec du DMF (4 × 2 min), du CH₂Cl₂ (6 × 2 min), de l'éther éthylique puis séchée sous pression réduite. La déprotection et la coupure des peptides sont ensuite réalisées avec une solution de TFA/H₂O/EDT/TIS : 94/2,5/2,5/1 en volume pour le peptide 1) et TFA/H₂O/TIS : 95/2,5/2,5 en volume pour le peptide 2) pendant 1h. Les peptides sont ensuite précipités dans une solution Et₂O/*n*-heptane : 1/1 en volume, solubilisés dans l'eau et lyophilisés. La purification est réalisée par RP-HPLC et on obtient 41 mg (20 %) et 28 mg (14 %) pour les peptides 1) et 2) respectivement.
MALDI-TOF (matrice DHB) Peptide 1) : Mcalc = 1505,7 (C₇₄H₁₀₄N₁₆O₁₆S) ; m/z = 1506,8 [M + H]⁺ ; m/z = 1528,8 [M + Na]⁺.
MALDI-TOF (matrice DHB) Peptide 2) : Mcalc = 1488,7 (C₇₅H₁₀₅N₁₅O₁₇) ; m/z = 1489,7 [M + H]⁺ ; m/z = 1511,7 [M + Na]⁺.

Les polymères **PsAcAr1** et **PsAcArF5** sont incubés dans une plaque 96-puits en polystyrène à une concentration de 10 µg/mL dans du PBS pendant une nuit à température ambiante sous agitation (100 µL / puits).

Les puits sont ensuite lavés 6 fois avec une solution de PBS / 0,1% Tween 20. La plaque est séchée à la centrifugeuse.

Les peptides 1) et 2) sont solubilisés à une concentration de 10⁻⁴ M dans du tampon phosphate à 200 mM en présence de 200 mM d'acide mercaptophénylacétique (MPAA), de 80 mM de tris(carboxyéthyl)phosphine, à pH 7,6. Ces solutions sont incubées pendant 10 min sous agitation à 37°C (60 µL / puits) dans les puits fonctionnalisés.

Les puits sont ensuite lavés 6 fois avec une solution de PBS / 0,1% Tween 20 et 3 fois à l'eau déionisée. La plaque est séchée à la centrifugeuse (2500 tours/min, 5 min, 20°C). La plaque est analysée au scanner par fluorescence Técan.

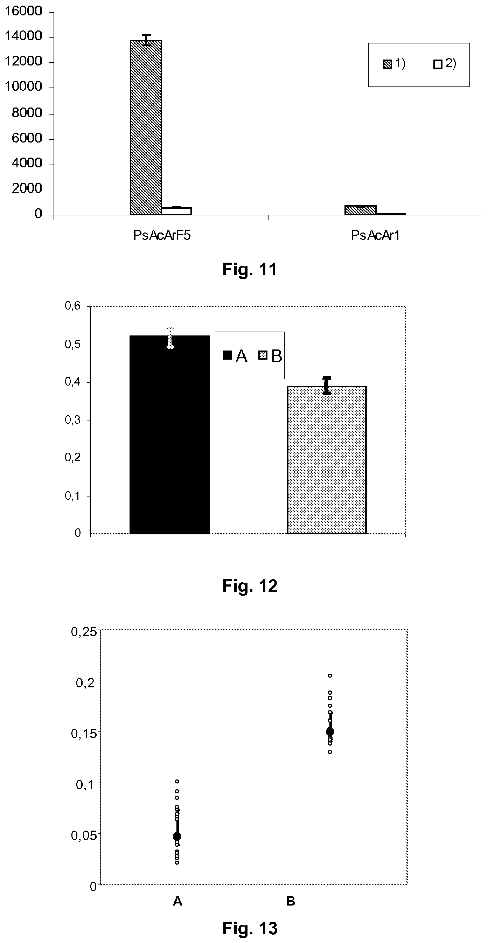

Les résultats sont représentés à la **figure 11** (intensité de fluorescence en ordonnée). Cet exemple démontre que les puits de la plaque polystyrène fonctionnalisée à l'aide du polymère **PsAcArF5** permettent la liaison du peptide cystéine 1) et non pas du peptide sérine témoin 2). Le signal très faible obtenu avec le polymère **PsAcAr1** témoin pour les deux peptides montre l'importance d'avoir une fonction 2,5-dithiazépane sur la surface polystyrène pour une liaison du peptide cystéine 1).

### Exemple 34 - préparation d'un biopuce à anticorps au fond de plaques à 96 puits en polystyrène

Dans cet exemple, les puits sont fonctionnalisés à l'aide d'un polysaccharide, puis les anticorps sont imprimés pour former une biopuce. Ces protéines se lient à la surface par la formation de liens non covalents. Les biopuces sont ensuite incubées avec des protéines cible.

8 puits d'une microplaque 96 puits en polystyrène (Maxisorp, Nunc-Immuno Plate) sont traités avec 100 µL de **PsAcArF1 (1,15 ; 0,64 ; 0,26)** à une concentration de 10 µg/mL dans un tampon (conditions A). 8 puits sont traités avec le tampon seul (conditions B). Le traitement est réalisé à température ambiante sous agitation pendant une nuit.

La plaque est ensuite lavée au tampon à l'aide d'un laveur de plaques (200 µL/puits, 3 lavages, agitation) puis à l'eau deionisée à l'aide d'un laveur de plaques (200 µL/puits, 3 lavages, agitation).

L'anticorps anti-streptavidine (anticorps polyclonal de lapin anti-streptavidine, Antibodies-on line, référence ABIN107091) est ensuite imprimé au fond des puits solubilisé dans le tampon (3 spots par puits, 8 puits soit au total 24 spots).

La saturation des puits est réalisée avec 200 µL de tampon avec 2% de BSA pendant 2h.

Les puits sont lavés au laveur automatique (3 cycles, agitation) avec du tampon additionné de 0,05 % de Tween 20.

On incube ensuite la streptavidine-HRP (Streptavidin-HRP, réference Thermo-Pierce 21130) avec une dilution 1:10.000 dans du tampon additionné de Tween 20 à 0,05% et BSA à 0,2% pendant 1h (100 µL/puits).

Une expérience supplémentaire est réalisée avec une protéine contrôle : goat anti-mouse IgG HRP conjugate, Southern Biotech, ref 103105, diluée 1:6000 dans la même solution.

Les puits sont lavés au laveur automatique (3 cycles) avec du tampon contenant 0,05% de Tween 20. On incube ensuite avec une solution de TMB (3,3', 5,5"-tétraméthylbenzidine) insoluble pendant 12 minutes à température ambiante. Les puits sont lavés avec 200 µL d'eau déionisée.

Une image du fond de chaque puits est aquise à l'aide d'une camera CCD (résolution : 3 µm) qui permet de générer des images en niveau de gris. Les spots sont ensuite quantifiés à l'aide d'un logiciel standard de quantification de microarrays.

Les résultats des intensités pour l'ensemble des spots après incubation avec la streptavidine-HRP sont reportés dans la **figure 12** (intensité en unités arbitraires en ordonnée ; A : puits traités avec **PsAcArF1 (1,15 ; 0,64 ; 0,26)** ; B : puits témoins non traités). L'incubation avec la protéine témoin goat anti-mouse IgG HRP conjugate n'a donné aucun signal détectable.

Cette figure montre que l'intensité médiane obtenue pour les spots d'anticorps anti-streptavidine au sein des puits A est significativement supérieure à celle des puits B.

Les résultats de la déviation standard des pixels pour chaque spot (ronds vides), avec la médiane et la déviation interquartile pour l'ensemble des spots sont reportés dans la **figure 13****.** Cette déviation standard au sein des spots, qui caractérise la qualité du spotting, est très nettement inférieure pour la condition A comparé à la condition B.

Globalement, ces résultats montrent la supériorité du traitement A pour la préparation de biopuces.

## Revendications

1. Dispositif de détection d'analytes, comprenant un substrat en matière plastique au moins en partie directement recouvert par des polymères de liaison fixés au substrat de manière non-covalente, lesdits polymères de liaison comportant un squelette polysaccharidique pourvu :
- de groupements aromatiques de la forme -X-CONH-Z, où X représente une chaîne alkyle linéaire ou ramifiée, substituée ou non, comprenant de 1 à 6 atomes de carbone, et Z représente une fonction aryle ; et
- de groupements acides carboxyliques de la forme -X-COOH, où X représente une chaîne alkyle linéaire ou ramifiée, substituée ou non, comprenant de 1 à 6 atomes de carbone ;
ledit dispositif étant **caractérisé en ce que** le squelette polysaccharidique est en outre pourvu de groupements réactifs F, lesdits groupements réactifs étant de la forme -X-CONH-Z', où X représente une chaîne alkyle linéaire ou ramifiée, substituée ou non, comprenant de 1 à 6 atomes de carbone, et Z' représente un groupement apte à se lier à une autre molécule, Z' étant choisi parmi :
- X'-N₃ ;
- NH₂ ; 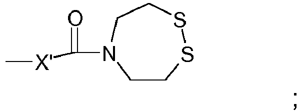 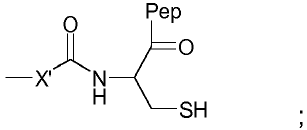 où X' représente une chaîne alkyle, substituée ou non, comprenant de 1 à 6 atomes de carbone et Pep représente un fragment peptidique.

2. Dispositif selon la revendication 1, dans lequel le squelette polysaccharidique est un squelette de dextran, dont le poids moléculaire est de préférence compris entre 15 000 et 100 000 Da, et de manière plus particulièrement préférée entre 30 000 et 60 000 Da.

3. Dispositif selon l'une des revendications 1 à 2, dans lequel :
- X est CH₂ ; et / ou
- Z est -CH₂-Ph ou -CH₂-Ph-*para*OH.

4. Dispositif selon l'une des revendications 1 à 3, dans lequel les polymères de liaison comportent :
- de 0,4 à 0,8 groupements aromatiques, de préférence de 0,4 à 0,6 groupements aromatiques, de préférence de 0,45 à 0,6 groupements aromatiques par unité saccharidique du squelette polysaccharidique ; et / ou
- de 0 à 0,8 groupements réactifs, de préférence de 0 à 0,6 groupements réactifs, par unité saccharidique du squelette polysaccharidique ; et / ou
- de 0,5 à 1,5 groupements aromatiques, acides carboxyliques et réactifs au total, de préférence de 0,9 à 1,3 groupements aromatiques, acides carboxyliques et réactifs au total, par unité saccharidique du squelette polysaccharidique.

5. Dispositif selon l'une des revendications 1 à 4, dans lequel le substrat est un substrat de polystyrène, polycarbonate, polyméthacrylate de méthyle ou polypropylène, et de préférence de polystyrène.

6. Dispositif selon l'une des revendications 1 à 5, comportant des éléments de capture immobilisés sur les polymères de liaison, l'immobilisation des éléments de capture sur les polymères de liaison étant de préférence réalisée par adsorption ou par liaison covalente aux groupements réactifs F.

7. Dispositif selon la revendication 6, dans lequel les éléments de capture sont choisis parmi :
- les polypeptides éventuellement modifiés et/ou conjugués ;
- les saccharides, les oligosaccharides et les lipopolysaccharides ;
- les virus ou fragments de virus et les cellules.

8. Dispositif selon l'une des revendications 1 à 7, comportant une pluralité de zones de détection, les zones de détection comportant de préférence des éléments de capture différents.

9. Dispositif selon l'une des revendications 1 à 8, dans lequel le substrat est une lame opaque ou transparente, une plaque de microtitration, un ensemble de billes, une plaque de culture, une bandelette ou un bâtonnet.

10. Utilisation d'un dispositif de détection d'analytes selon l'une des revendications 1 à 9, pour la détection et éventuellement la quantification de molécules chimiques, de molécules biologiques, de cellules ou d'organismes vivants.

11. Polymère de liaison susceptible d'être utilisé dans le dispositif selon l'une quelconque des revendications 1 à 9 comportant un squelette polysaccharidique pourvu :
- de 0,4 à 0,8 groupements aromatique par unité saccharidique de la forme -X-CONH-Z, où X représente une chaîne alkyle linéaire ou ramifiée, substituée ou non, comprenant de 1 à 6 atomes de carbone, et Z représente une fonction aryle ;
- des groupements acides carboxyliques de la forme -X-COOH, où X représente une chaîne alkyle linéaire ou ramifiée, comprenant de 1 à 6 atomes de carbone,
- de groupements réactifs F de la forme -X-CONH-Z', où X représente une chaîne alkyle linéaire ou ramifiée, substituée ou non, comprenant de 1 à 6 atomes de carbone, et Z' est choisi parmi :
-X'-N₃ ;
-NH₂ ; 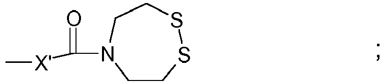 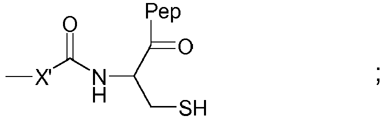 où X' représente une chaîne alkyle, substitué ou non, comprenant de 1 à 6 atomes de carbone et Pep représente un fragment peptidique.

12. Polymère de liaison selon la revendication précédente, dans lequel le squelette polysaccharidique est un squelette de dextran, dont le poids moléculaire est de préférence compris entre 15 000 et 100 000 Da, et de manière plus particulièrement préférée entre 30 000 et 60 000 Da.

13. Polymère de liaison selon l'une des revendications 11 à 12, dans lequel :
- X et/ou X' est CH₂ ; et/ou
- X et/ou X' est (CH₂)₂ ; et/ou
- Z est -CH₂-Ph ou -CH₂-Ph-*para*OH.

14. Polymère de liaison selon l'une des revendications 11 à 13, comprenant :
- de 0,4 à 0,6 groupements aromatiques, de préférence de 0,45 à 0,6 groupements aromatiques par unité saccharidique du squelette polysaccharidique ; et / ou
- jusqu'à 0,8 groupements réactifs, de préférence de jusqu'à 0,6 groupements réactifs, par unité saccharidique du squelette polysaccharidique ; et / ou
- de 0,5 à 1,5 groupements aromatiques, acides carboxyliques et réactifs au total, de préférence de 0,9 à 1,3 groupements aromatiques, acides carboxyliques et réactifs au total, par unité saccharidique du squelette polysaccharidique.

## Patentansprüche

1. Vorrichtung zur Detektion von Analyten, umfassend ein Substrat aus Kunststoffmaterial, das mindestens teilweise mit Bindungspolymeren bedeckt ist, die an dem Substrat auf nicht-kovalente Weise fixiert sind, wobei die Bindungspolymere ein Polysaccharid-Gerüst umfassen, das versehen ist mit:
- aromatischen Gruppen mit der Form -X-CONH-Z, wobei X eine lineare oder verzweigte, substituierte oder nicht substituierte Alkylkette darstellt, umfassend 1 bis 6 Kohlenstoffatome, und Z eine Arylfunktion darstellt; und
- Carbonsäuregruppen mit der Form -X-COOH, wobei X eine lineare oder verzweigte, substituierte oder nicht substituierte Alkylkette darstellt, umfassend 1 bis 6 Kohlenstoffatome;
wobei die Vorrichtung **dadurch gekennzeichnet ist, dass** das Polysaccharid-Gerüst außerdem mit reaktiven Gruppen F versehen ist, wobei die reaktiven Gruppen die Form -X-CONH-Z' aufweisen, wobei X eine lineare oder verzweigte, substituierte oder nicht substituierte Alkylkette darstellt, umfassend 1 bis 6 Kohlenstoffatome, und Z' eine Gruppe darstellt, die dafür geeignet ist, sich an ein anderes Molekül zu binden, wobei Z' ausgewählt ist aus:
- X'-N₃;
- NH₂; 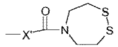 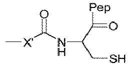 wobei X' eine substituierte oder nicht substituierte Alkylkette darstellt, umfassend 1 bis 6 Kohlenstoffatome, und Pep ein Peptidfragment darstellt.

2. Vorrichtung nach Anspruch 1, wobei das Polysaccharid-Gerüst ein Dextran-Gerüst ist, dessen Molekulargewicht vorzugsweise zwischen 15 000 und 100 000 Da, und besonders bevorzugt zwischen 30 000 und 60 000 Da beträgt.

3. Vorrichtung nach einem der Ansprüche 1 bis 2, wobei:
- X CH₂ darstellt; und/oder
- Z -CH₂-Ph oder -CH₂-Ph-*para*OH ist.

4. Vorrichtung nach einem der Ansprüche 1 bis 3, wobei die Bindungspolymere umfassen:
- von 0,4 bis 0,8 aromatische Gruppen, vorzugsweise von 0,4 bis 0,6 aromatische Gruppen, vorzugsweise von 0,45 bis 0,6 aromatische Gruppen pro Saccharid-Einheit des Polysaccharid-Gerüsts; und/oder
- von 0 bis 0,8 reaktive Gruppen, vorzugsweise von 0 bis 0,6 reaktive Gruppen, pro Saccharid-Einheit des Polysaccharid-Gerüsts; und/oder
- insgesamt von 0,5 bis 1,5 aromatische, Carbonsäure- und reaktive Gruppen, vorzugsweise insgesamt von 0,9 bis 1,3 aromatische, Carbonsäure- und reaktive Gruppen, pro Saccharid-Einheit des Polysaccharid-Gerüsts.

5. Vorrichtung nach einem der Ansprüche 1 bis 4, wobei das Substrat ein Substrat aus Polystyrol, Polycarbonat, Polymethylmethacrylat oder Polypropylen, und vorzugsweise aus Polystyrol ist.

6. Vorrichtung nach einem der Ansprüche 1 bis 5, umfassend Einfangelemente, die auf den Bindungspolymeren immobilisiert sind, wobei die Immobilisation der Einfangselemente auf den Bindungspolymeren vorzugsweise durch Adsorption oder durch kovalente Bindung an reaktive Gruppen F durchgeführt wird.

7. Vorrichtung nach Anspruch 6, wobei die Einfangelemente ausgewählt sind aus:
- gegebenenfalls modifizierten und/oder konjugierten Polypeptiden;
- Sacchariden, Oligosacchariden und Lipopolysacchariden;
- Viren oder Virusfragmenten und Zellen.

8. Vorrichtung nach einem der Ansprüche 1 bis 7, umfassend eine Vielzahl von Detektionszonen, wobei die Detektionszonen vorzugsweise verschiedene Einfangelemente umfassen.

9. Vorrichtung nach einem der Ansprüche 1 bis 8, wobei das Substrat eine opake oder transparente Lamelle, eine Mikrotitrationsplatte, eine Anordnung von Kügelchen, eine Kulturplatte, ein Band oder ein Streifen ist.

10. Verwendung einer Vorrichtung zur Detektion von Analyten nach einem der Ansprüche 1 bis 9, für die Detektion und gegebenenfalls die Quantifizierung von chemischen Molekülen, biologischen Molekülen, Zellen oder lebenden Organismen.

11. Bindungspolymer, welches in der Vorrichtung nach einem der Ansprüche 1 bis 9 verwendet werden kann, umfassend ein Polysaccharid-Gerüst, das versehen ist mit:
- von 0,4 bis 0,8 aromatischen Gruppen pro Saccharid-Einheit mit der Form -X-CONH-Z, wobei X eine lineare oder verzweigte, substituierte oder nicht substituierte Alkylkette darstellt, umfassend 1 bis 6 Kohlenstoffatome, und Z eine Arylfunktion darstellt;
- Carbonsäuregruppen mit der Form -X-COOH, wobei X eine lineare oder verzweigte Alkylkette darstellt, umfassend 1 bis 6 Kohlenstoffatome;
- reaktiven Gruppen F mit der Form -X-CONH-Z', wobei X eine lineare oder verzweigte, substituierte oder nicht substituierte Alkylkette darstellt, umfassend 1 bis 6 Kohlenstoffatome, und Z' ausgewählt ist aus:
- X'-N₃;
- NH₂; 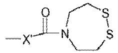 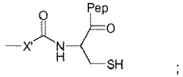 wobei X' eine substituierte oder nicht substituierte Alkylkette darstellt, umfassend 1 bis 6 Kohlenstoffatome, und Pep ein Peptidfragment darstellt.

12. Bindungspolymer nach dem vorhergehenden Anspruch, wobei das Polysaccharid-Gerüst ein Dextran-Gerüst ist, dessen Molekulargewicht vorzugsweise zwischen 15 000 und 100 000 Da, und besonders bevorzugt zwischen 30 000 und 60 000 Da beträgt.

13. Bindungspolymer nach einem der Ansprüche 11 oder 12, wobei:
- X und/oder X' CH₂ darstellt; und/oder
- X und/oder X' (CH₂)₂ darstellt; und/oder
- Z -CH₂-Ph oder -CH₂-Ph-*para*OH ist.

14. Bindungspolymer nach einem der Ansprüche 11 bis 13, umfassend:
- von 0,4 bis 0,6 aromatische Gruppen, vorzugsweise von 0,45 bis 0,6 aromatische Gruppen pro Saccharid-Einheit des Polysaccharid-Gerüsts; und/oder
- bis zu 0,8 reaktive Gruppen, vorzugsweise bis zu 0,6 reaktive Gruppen pro Saccharid-Einheit des Polysaccharid-Gerüsts; und/oder
- insgesamt von 0,5 bis 1,5 aromatische, Carbonsäure- und reaktive Gruppen, vorzugsweise insgesamt von 0,9 bis 1,3 aromatische, Carbonsäure- und reaktive Gruppen, pro Saccharid-Einheit des Polysaccharid-Gerüsts.

## Claims

1. Device for detecting analytes, comprising a plastic substrate at least partly covered directly with binding polymers fixed to the substrate non-covalently, said binding polymers comprising a polysaccharide skeleton provided with:
- aromatic groups of the form -X-CONH-Z, where X represents a linear or branched, substituted or unsubstituted alkyl chain, comprising 1 to 6 carbon atoms, and Z represents an aryl function;
- carboxylic acid groups of the form -X-COOH, where X represents a linear or branched, substituted or unsubstituted alkyl chain, comprising 1 to 6 carbon atoms
said device being **characterized in that** the polysaccharide skeleton further comprises reactive F groups, said reactive groups being of the form -X-CONH-Z', where X represents a linear or branched, substituted or unsubstituted alkyl chain, comprising 1 to 6 carbon atoms, and Z' represents a group capable of binding to another molecule, Z' being selected among:
-X'-N₃;
-NH₂; 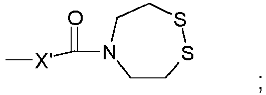 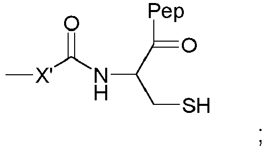 where X' represents a substituted or unsubstituted alkyl chain comprising from 1 to 6 carbon atoms and Pep represents a peptide fragment.

2. Device according to claim 1, in which the polysaccharide skeleton is a dextran skeleton, the molecular weight of which is preferably between 15 000 and 100 000 Da, and more particularly preferably between 30 000 and 60 000 Da.

3. Device according to claim 1 or 2, in which:
- X is CH₂; and/or
- Z is -CH₂-Ph or -CH₂-Ph-*para*OH.

4. Device according to one of claims 1 to 3, in which the binding polymers comprise:
- from 0.4 to 0.8 aromatic groups, preferably from 0.4 to 0.6 aromatic groups, preferably from 0.45 to 0.6 aromatic groups per saccharide unit of the polysaccharide skeleton; and/or
- from 0 to 0.8 reactive groups, preferably from 0 to 0.6 reactive groups, per saccharide unit of the polysaccharide skeleton; and/or
- from 0.5 to 1.5 aromatic, carboxylic acid and reactive groups in total, preferably from 0.9 to 1.3 aromatic, carboxylic acid and reactive groups in total, per saccharide unit of the polysaccharide skeleton.

5. Device according to one of claims 1 to 4, in which the substrate is a substrate of polystyrene, polycarbonate, poly(methyl methacrylate) or polypropylene, and preferably of polystyrene.

6. Device according to one of claims 1 to 5, comprising capturing elements immobilized on the binding polymers, said capturing elements preferably being immobilized on the binding polymers by adsorption or by covalent bonding to the reactive F groups.

7. Device according to claim 6, in which the capturing elements are selected from:
- polypeptides, optionally modified and/or conjugated;
- saccharides, oligosaccharides and lipopolysaccharides;
- viruses or virus fragments and cells.

8. Device according to one of claims 1 to 7, comprising a plurality of detection zones, the detection zones preferably comprising different capturing elements.

9. Device according to one of claims 1 to 8, in which the substrate is an opaque or transparent slide, a microtitre plate, a collection of beads, a culture plate, a strip or a stick.

10. Use of a device for detecting analytes according to one of claims 1 to 9, for detecting and optionally quantifying chemical molecules, biological molecules, cells or living organisms.

11. Binding polymer that can be used in the device according to any one of claims 1 to 9 comprising a polysaccharide skeleton provided with:
- 0.4 to 0.8 aromatic groups per saccharide unit of the form -X-CONH-Z, where X represents a linear or branched, substituted or unsubstituted alkyl chain, comprising 1 to 6 carbon atoms, and Z represents an aryl function;
- carboxylic acid groups of the form -X-COOH, where X represents a linear or branched alkyl chain, comprising 1 to 6 carbon atoms;
- reactive F groups being of the form -X-CONH-Z', where X represents a linear or branched, substituted or unsubstituted alkyl chain, comprising 1 to 6 carbon atoms, and Z' being selected among:
-X'-N₃;
-NH₂; 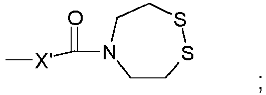 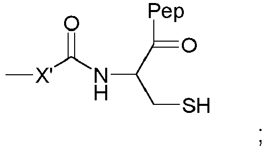 where X' represents a substituted or unsubstituted alkyl chain comprising from 1 to 6 carbon atoms and Pep represents a peptide fragment.

12. Binding polymer according to the preceding claim, in which the polysaccharide skeleton is a dextran skeleton, the molecular weight of which is preferably between 15 000 and 100 000 Da, and more particularly preferably between 30 000 and 60 000 Da.

13. Binding polymer according to one of claims 11 to 12 , in which:
- X and/or X' is CH₂; and/or
- X and/or X' is (CH₂)₂; and/or
- Z is -CH₂-Ph or -CH₂-Ph-*para*OH.

14. Binding polymer according to one of claims 11 to 13, comprising:
- from 0.4 to 0.6 aromatic groups, preferably from 0.45 to 0.6 aromatic groups, per saccharide unit of the polysaccharide skeleton; and/or
- from 0 to 0.8 reactive groups, preferably from 0 to 0.6 reactive groups, per saccharide unit of the polysaccharide skeleton; and/or
- from 0.5 to 1.5 aromatic, carboxylic acid and reactive groups in total, preferably from 0.9 to 1.3 aromatic, carboxylic acid and reactive groups in total, per saccharide unit of the polysaccharide skeleton.
